(19)

Europäisches
Patentamt
European
Patent Office
Office européen
des brevets

(11) **EP 3 928 702 A2**

(12) **EUROPEAN PATENT APPLICATION**

(43) Date of publication:
**29.12.2021 Bulletin 2021/52**

(51) Int Cl.:
***A61B 5/361*** (2021.01)   ***A61B 5/00*** (2006.01)
***A61B 5/367*** (2021.01)

(21) Application number: **21177845.1**

(22) Date of filing: **04.06.2021**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.06.2020   US 202063034508 P
03.06.2021   US 202117337584**

(71) Applicant: **Biosense Webster (Israel) Ltd
Yokneam, 2066717 (IL)**

(72) Inventors:
• **DORON,, Itai
  2066717 Yokneam (IL)**
• **ZIV-ARI,, Morris
  2066717 Yokneam (IL)**
• **COHEN,, Assaf
  2066717 Yokneam (IL)**

(74) Representative: **Carpmaels & Ransford LLP
One Southampton Row
London WC1B 5HA (GB)**

(54) **ECG BASED ARRHYTHMIA LOCATION IDENTIFICATION AND IMPROVED MAPPING**

(57)     Systems, devices, and techniques are disclosed for automatically detecting arrhythmia locations. The systems, devices, and techniques include a plurality of body surface electrodes configured to sense electrocardiogram (ECG) data. The systems, devices, and techniques include a processor including a neural network configured to receive a plurality of historical ECG data and corresponding arrhythmia locations determined based on each of the plurality of historical ECG data, train a learning system based on the plurality of historical ECG data and corresponding arrhythmia locations, generate a model based on the learning system. New ECG data may be received from the plurality of body surface electrodes and the processor may provide a new arrhythmia location based on the new ECG data. Additionally, a new coherent mapping adjustment may be provided based on a model that is trained using historical coherent mapping adjustments.

FIG. 4

**Description**

CROSS REFERENCE TO RELATED APPLICATIONS

This application claims the benefit of United States Provisional Patent Application No. 63/034,508 (JNJBIO-6322USPSP1) filed on June 4, 2020, which is incorporated by reference as if fully set forth.

FIELD OF INVENTION

The present teachings are related to artificial intelligence and machine learning associated with optimizing mapping and identifying optimal areas for conducting cardiac procedure.

BACKGROUND

Medical conditions, such as cardiac arrhythmia (e.g., atrial fibrillation (AF)), are often diagnosed and treated via intra-body procedures. For example, electrical pulmonary vein isolation (PVI) from the left atrial (LA) body is performed using ablation for treating AF. PVI, and many other minimally invasive catheterizations, require real-time visualization and mapping of an intra-body surface.

Visualization and mapping of intra-body body parts can be performed by mapping propagation of activation waves, Fluoroscopies, computerized tomography (CT) and magnetic resonance imaging (MRI), as well as other techniques which may require a greater than desirable amount of time or resources to provide the visualization and mapping.

Additionally, medical professionals often observe an electrocardiogram (ECG) in an attempt to locate the site of an arrhythmia. Successfully identifying such an arrhythmia can often require years of medical training and can still result in human error.

SUMMARY

Systems, devices, and techniques are disclosed for automatically detecting arrhythmia locations. The systems, devices, and techniques may include a plurality of body surface electrodes configured to sense electrocardiogram (ECG) data. The systems, devices, and techniques may include a processor, including a neural network, configured to receive a plurality of historical ECG data and corresponding arrhythmia locations determined based on each of the plurality of historical ECG data, train a learning system based on the plurality of historical ECG data and corresponding arrhythmia locations, and generate a model based on the learning system. New ECG data may be received from the plurality of body surface electrodes, and the processor may provide a new arrhythmia location based on the new ECG data. Additionally, a new coherent mapping adjustment may be provided based on a model that is trained using historical coherent mapping adjustments.

BRIEF DESCRIPTION OF THE DRAWINGS

A more detailed understanding may be had from the following description, given by way of example in conjunction with the accompanying drawings, wherein like reference numerals in the figures indicate like elements, and wherein:

FIG. 1 is a block diagram of an example system for remotely monitoring and communicating patient biometrics;
FIG. 2 is a system diagram of an example of a computing environment in communication with network;
FIG. 3 is a block diagram of an example device in which one or more features of the disclosure can be implemented;
FIG. 4 illustrates a graphical depiction of an artificial intelligence system incorporating the example device of FIG. 3;
FIG. 5 illustrates a method performed in the artificial intelligence system of FIG. 4;
FIG. 6 illustrates an example of the probabilities of a naive Bayes calculation;
FIG. 7 illustrates an exemplary decision tree;
FIG. 8 illustrates an exemplary random forest classifier;
FIG. 9 illustrates an exemplary logistic regression;
FIG. 10 illustrates an exemplary support vector machine;
FIG. 11 illustrated an exemplary linear regression model;
FIG. 12 illustrates an exemplary K-means clustering;
FIG. 13 illustrates an exemplary ensemble learning algorithm;
FIG. 14 illustrates an exemplary neural network;
FIG. 15 illustrates a hardware based neural network;
FIGs. 16A through 16D show examples of cardiomyopathies with different etiologies;

FIG. 17 is a diagram of an exemplary system in which one or more features of the disclosure subject matter can be implemented;

FIG. 18A shows an example of a linear catheter including multiple electrodes;

FIG. 18B shows an example of a balloon catheter including multiple splines;

FIG. 18C shows an example of a loop catheter including multiple electrodes;

FIG. 19 is a flowchart for identifying cardiac locations based on ECG data and a model;

FIG. 20A shows example ECG data and related cardiac locations;

FIG. 20B shows another example ECG data and related cardiac locations;

FIG. 21 shows an example logistic regression diagram to predict whether certain ECG characteristics are likely to correspond to a given cardiac location with an arrhythmia;

FIG. 22 shows a flowchart for applying coherent mapping adjustments based on patient specific data; and

FIGs. 23A-C show inherent limitations related to chamber reconstruction and data projection.

## DETAILED DESCRIPTION

According to exemplary embodiments of the disclosed subject matter, a medical procedure may be optimized by applying historical electrocardiogram (ECG) data that is successfully used to map the location of an arrhythmia to predict the location of an arrhythmia such that the arrhythmia can be treated.

Additionally, according to exemplary embodiments of the disclosed subject matter cardiac mapping/renderings may be improved based on a number of factors in addition to location-based mapping. Such factors may be improved based on an evaluation of a given patient and the corresponding mapping over a period of time (e.g., 30 seconds) during which unwanted externalities are corrected from a given cardiac mapping. Such unwanted externalities can be, but are not limited to, noise, cyclical data (e.g., respiration), position correction, and the like. As disclosed herein, training data may include a plurality of such corrections may be provided such that a given model is trained to automatically correct such unwanted externalities for a new cardiac mapping, without requiring the evaluation of a new patient over a period of time (e.g., the 30 seconds).

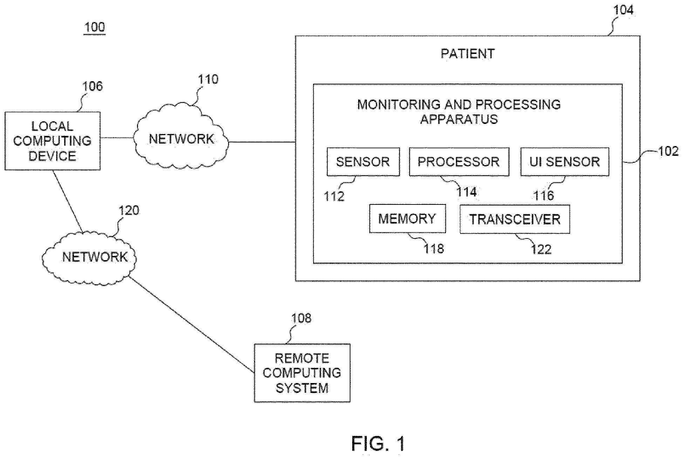

FIG. 1 is a block diagram of an example system 100 for remotely monitoring and communicating patient biometrics (i.e., patient data). In the example illustrated in FIG. 1, the system 100 includes a patient biometric monitoring and processing apparatus 102 associated with a patient 104, a local computing device 106, a remote computing system 108, a first network 110 and a second network 120.

According to an embodiment, a monitoring and processing apparatus 102 may be an apparatus that is internal to the patient's body (e.g., subcutaneously implantable). The monitoring and processing apparatus 102 may be inserted into a patient via any applicable manner including orally injecting, surgical insertion via a vein or artery, an endoscopic procedure, or a laparoscopic procedure.

According to an embodiment, a monitoring and processing apparatus 102 may be an apparatus that is external to the patient. For example, as described in more detail below, the monitoring and processing apparatus 102 may include an attachable patch (e.g., that attaches to a patient's skin). The monitoring and processing apparatus 102 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an embodiment, a monitoring and processing apparatus 102 may include both components that are internal to the patient and components that are external to the patient.

A single monitoring and processing apparatus 102 is shown in FIG. 1. Example systems may, however, may include a plurality of patient biometric monitoring and processing apparatuses. A patient biometric monitoring and processing apparatus may be in communication with one or more other patient biometric monitoring and processing apparatuses. Additionally, or alternatively, a patient biometric monitoring and processing apparatus may be in communication with the network 110.

One or more monitoring and processing apparatuses 102 may acquire patient biometric data (e.g., electrical signals, blood pressure, temperature, blood glucose level or other biometric data) and receive at least a portion of the patient biometric data representing the acquired patient biometrics and additional formation associated with acquired patient biometrics from one or more other monitoring and processing apparatuses 102. The additional information may be, for example, diagnosis information and/or additional information obtained from an additional device such as a wearable device. Each monitoring and processing apparatus 102 may process data, including its own acquired patient biometrics as well as data received from one or more other monitoring and processing apparatuses 102.

In FIG. 1, network 110 is an example of a short-range network (e.g., local area network (LAN), or personal area network (PAN)). Information may be sent, via short-range network 110, between monitoring a processing apparatus 102 and local computing device 106 using any one of various short-range wireless communication protocols, such as Bluetooth, Wi-Fi, Zigbee, Z-Wave, near field communications (NFC), ultraband, Zigbee, or infrared (IR).

Network 120 may be a wired network, a wireless network or include one or more wired and wireless networks. For example, a network 120 may be a long-range network (e.g., wide area network (WAN), the internet, or a cellular network,). Information may be sent, via network 120 using any one of various long-range wireless communication protocols (e.g., TCP/IP, HTTP, 3G, 4G/LTE, or 5G/New Radio).

The patient monitoring and processing apparatus 102 may include a patient biometric sensor 112, a processor 114, a user input (UI) sensor 116, a memory 118, and a transmitter-receiver (i.e., transceiver) 122. The patient monitoring and processing apparatus 102 may continually or periodically monitor, store, process and communicate, via network 110, any number of various patient biometrics. Examples of patient biometrics include electrical signals (e.g., ECG signals and brain biometrics), blood pressure data, blood glucose data and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

Patient biometric sensor 112 may include, for example, one or more sensors configured to sense a type of biometric patient biometrics. For example, patient biometric sensor 112 may include an electrode configured to acquire electrical signals (e.g., heart signals, brain signals or other bioelectrical signals), a temperature sensor, a blood pressure sensor, a blood glucose sensor, a blood oxygen sensor, a pH sensor, an accelerometer and a microphone.

As described in more detail below, patient biometric monitoring and processing apparatus 102 may be an ECG monitor for monitoring ECG signals of a heart. The patient biometric sensor 112 of the ECG monitor may include one or more electrodes for acquiring ECG signals. The ECG signals may be used for treatment of various cardiovascular diseases.

In another example, the patient biometric monitoring and processing apparatus 102 may be a continuous glucose monitor (CGM) for continuously monitoring blood glucose levels of a patient on a continual basis for treatment of various diseases, such as type I and type II diabetes. The CGM may include a subcutaneously disposed electrode, which may monitor blood glucose levels from interstitial fluid of the patient. The CGM may be, for example, a component of a closed-loop system in which the blood glucose data is sent to an insulin pump for calculated delivery of insulin without user intervention.

Transceiver 122 may include a separate transmitter and receiver. Alternatively, transceiver 122 may include a transmitter and receiver integrated into a single device.

Processor 114 may be configured to store patient data, such as patient biometric data in memory 118 acquired by patient biometric sensor 112, and communicate the patient data, across network 110, via a transmitter of transceiver 122. Data from one or more other monitoring and processing apparatus 102 may also be received by a receiver of transceiver 122, as described in more detail below.

According to an embodiment, the monitoring and processing apparatus 102 includes UI sensor 116 which may be, for example, a piezoelectric sensor or a capacitive sensor configured to receive a user input, such as a tapping or touching. For example, UI sensor 116 may be controlled to implement a capacitive coupling, in response to tapping or touching a surface of the monitoring and processing apparatus 102 by the patient 104. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface such that the tapping or touching of the surface activates the monitoring device.

As described in more detail below, the processor 114 may be configured to respond selectively to different tapping patterns of the capacitive sensor (e.g., a single tap or a double tap), which may be the UI sensor 116, such that different tasks of the patch (e.g., acquisition, storing, or transmission of data) may be activated based on the detected pattern. In some embodiments, audible feedback may be given to the user from processing apparatus 102 when a gesture is detected.

The local computing device 106 of system 100 is in communication with the patient biometric monitoring and processing apparatus 102 and may be configured to act as a gateway to the remote computing system 108 through the second network 120. The local computing device 106 may be, for example, a, smart phone, smartwatch, tablet or other portable smart device configured to communicate with other devices via network 120. Alternatively, the local computing device 106 may be a stationary or standalone device, such as a stationary base station including, for example, modem and/or router capability, a desktop or laptop computer using an executable program to communicate information between the processing apparatus 102 and the remote computing system 108 via the PC's radio module, or a USB dongle. Patient biometrics may be communicated between the local computing device 106 and the patient biometric monitoring and processing apparatus 102 using a short-range wireless technology standard (e.g., Bluetooth, Wi-Fi, ZigBee, Z-wave and other short-range wireless standards) via the short-range wireless network 110, such as a local area network (LAN) (e.g., a personal area network (PAN)). In some embodiments, the local computing device 106 may also be configured to display the acquired patient electrical signals and information associated with the acquired patient electrical signals, as described in more detail below.

In some embodiments, remote computing system 108 may be configured to receive at least one of the monitored patient biometrics and information associated with the monitored patient via network 120, which is a long-range network. For example, if the local computing device 106 is a mobile phone, network 120 may be a wireless cellular network, and information may be communicated between the local computing device 106 and the remote computing system 108 via a wireless technology standard, such as any of the wireless technologies mentioned above. As described in more detail below, the remote computing system 108 may be configured to provide (e.g., visually display and/or aurally provide) the at least one of the patient biometrics and the associated information to a healthcare professional (e.g., a physician).

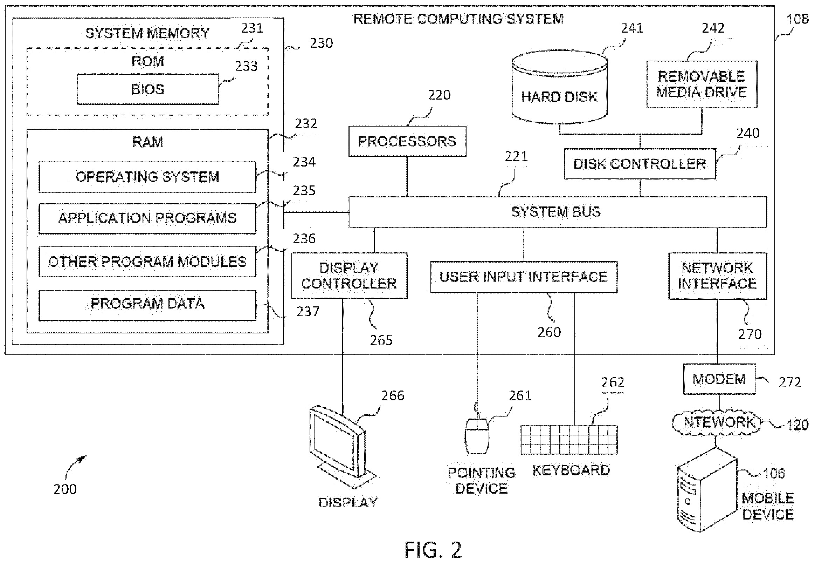

FIG. 2 is a system diagram of an example of a computing environment 200 in communication with network 120. In some instances, the computing environment 200 is incorporated in a public cloud computing platform (such as Amazon Web Services or Microsoft Azure), a hybrid cloud computing platform (such as HP Enterprise OneSphere) or a private cloud computing platform.

As shown in FIG. 2, computing environment 200 includes remote computing system 108 (hereinafter computer system), which is one example of a computing system upon which embodiments described herein may be implemented.

The remote computing system 108 may, via processors 220, which may include one or more processors, perform various functions. The functions may include analyzing monitored patient biometrics and the associated information and, according to physician-determined or algorithm driven thresholds and parameters, providing (e.g., via display 266) alerts, additional information or instructions. As described in more detail below, the remote computing system 108 may be used to provide (e.g., via display 266) healthcare personnel (e.g., a physician) with a dashboard of patient information, such that such information may enable healthcare personnel to identify and prioritize patients having more critical needs than others.

As shown in FIG. 2, the computer system 210 may include a communication mechanism such as a bus 221 or other communication mechanism for communicating information within the computer system 210. The computer system 210 further includes one or more processors 220 coupled with the bus 221 for processing the information. The processors 220 may include one or more CPUs, GPUs, or any other processor known in the art.

The computer system 210 also includes a system memory 230 coupled to the bus 221 for storing information and instructions to be executed by processors 220. The system memory 230 may include computer readable storage media in the form of volatile and/or nonvolatile memory, such as read only system memory (ROM) 231 and/or random-access memory (RAM) 232. The system memory RAM 232 may include other dynamic storage device(s) (e.g., dynamic RAM, static RAM, and synchronous DRAM). The system memory ROM 231 may include other static storage device(s) (e.g., programmable ROM, erasable PROM, and electrically erasable PROM). In addition, the system memory 230 may be used for storing temporary variables or other intermediate information during the execution of instructions by the processors 220. A basic input/output system 233 (BIOS) may contain routines to transfer information between elements within computer system 210, such as during start-up, that may be stored in system memory ROM 231. RAM 232 may comprise data and/or program modules that are immediately accessible to and/or presently being operated on by the processors 220. System memory 230 may additionally include, for example, operating system 234, application programs 235, other program modules 236 and program data 237.

The illustrated computer system 210 also includes a disk controller 240 coupled to the bus 221 to control one or more storage devices for storing information and instructions, such as a magnetic hard disk 241 and a removable media drive 242 (e.g., floppy disk drive, compact disc drive, tape drive, and/or solid-state drive). The storage devices may be added to the computer system 210 using an appropriate device interface (e.g., a small computer system interface (SCSI), integrated device electronics (IDE), Universal Serial Bus (USB), or FireWire).

The computer system 210 may also include a display controller 265 coupled to the bus 221 to control a monitor or display 266, such as a cathode ray tube (CRT) or liquid crystal display (LCD), for displaying information to a computer user. The illustrated computer system 210 includes a user input interface 260 and one or more input devices, such as a keyboard 262 and a pointing device 261, for interacting with a computer user and providing information to the processor 220. The pointing device 261, for example, may be a mouse, a trackball, or a pointing stick for communicating direction information and command selections to the processor 220 and for controlling cursor movement on the display 266. The display 266 may provide a touch screen interface that may allow input to supplement or replace the communication of direction information and command selections by the pointing device 261 and/or keyboard 262.

The computer system 210 may perform a portion or each of the functions and methods described herein in response to the processors 220 executing one or more sequences of one or more instructions contained in a memory, such as the system memory 230. Such instructions may be read into the system memory 230 from another computer readable medium, such as a hard disk 241 or a removable media drive 242. The hard disk 241 may contain one or more data stores and data files used by embodiments described herein. Data store contents and data files may be encrypted to improve security. The processors 220 may also be employed in a multiprocessing arrangement to execute the one or more sequences of instructions contained in system memory 230. In alternative embodiments, hard-wired circuitry may be used in place of or in combination with software instructions. Thus, embodiments are not limited to any specific combination of hardware circuitry and software.

As stated above, the computer system 210 may include at least one computer readable medium or memory for holding instructions programmed according to embodiments described herein and for containing data structures, tables, records, or other data described herein. The term computer readable medium as used herein refers to any non-transitory, tangible medium that participates in providing instructions to the processor 220 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 241 or removable media drive 242. Non-limiting examples of volatile media include dynamic memory, such as system memory 230. Non-limiting examples of transmission media include coaxial cables, copper wire, and fiber optics, including the wires that make up the bus 221. Transmission media may also take the form of acoustic or light waves, such as those generated during radio wave and infrared data communications.

The computing environment 200 may further include the computer system 210 operating in a networked environment using logical connections to local computing device 106 and one or more other devices, such as a personal computer (laptop or desktop), mobile devices (e.g., patient mobile devices), a server, a router, a network PC, a peer device or other common network node, and typically includes many or all of the elements described above relative to computer system 210. When used in a networking environment, computer system 210 may include modem 272 for establishing communications over a network 120, such as the Internet. Modem 272 may be connected to system bus 221 via network interface 270, or via another appropriate mechanism.

Network 120, as shown in FIGs. 1 and 2, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 610 and other computers (e.g., local computing device 106).

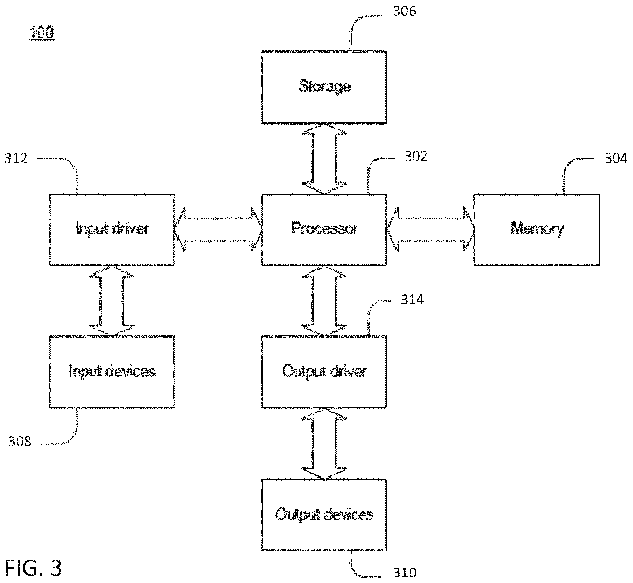

FIG. 3 is a block diagram of an example device 300 in which one or more features of the disclosure can be implemented. The device 300 may be local computing device 106, for example. The device 300 can include, for example, a computer, a gaming device, a handheld device, a set-top box, a television, a mobile phone, or a tablet computer. The device 300 includes a processor 302, a memory 304, a storage device 306, one or more input devices 308, and one or more output devices 310. The device 300 can also optionally include an input driver 312 and an output driver 314. It is understood that the device 300 can include additional components not shown in FIG. 3 including an artificial intelligence accelerator.

In various alternatives, the processor 302 includes a central processing unit (CPU), a graphics processing unit (GPU), a CPU and GPU located on the same die, or one or more processor cores, wherein each processor core can be a CPU or a GPU. In various alternatives, the memory 304 is located on the same die as the processor 302, or is located separately from the processor 302. The memory 304 includes a volatile or non-volatile memory, for example, random access memory (RAM), dynamic RAM, or a cache.

The storage device 306 includes a fixed or removable storage means, for example, a hard disk drive, a solid-state drive, an optical disk, or a flash drive. The input devices 308 include, without limitation, a keyboard, a keypad, a touch screen, a touch pad, a detector, a microphone, an accelerometer, a gyroscope, a biometric scanner, or a network connection (e.g., a wireless local area network card for transmission and/or reception of wireless IEEE 802 signals). The output devices 310 include, without limitation, a display, a speaker, a printer, a haptic feedback device, one or more lights, an antenna, or a network connection (e.g., a wireless local area network card for transmission and/or reception of wireless IEEE 802 signals).

The input driver 312 communicates with the processor 302 and the input devices 308, and permits the processor 302 to receive input from the input devices 308. The output driver 314 communicates with the processor 302 and the output devices 310, and permits the processor 302 to send output to the output devices 310. It is noted that the input driver 312 and the output driver 314 are optional components, and that the device 300 will operate in the same manner if the input driver 312 and the output driver 314 are not present. The output driver 316 includes an accelerated processing device ("APD") 316 which is coupled to a display device 318. The APD accepts compute commands and graphics rendering commands from processor 302, processes those compute and graphics rendering commands, and provides pixel output to display device 318 for display. As described in further detail below, the APD 316 includes one or more parallel processing units to perform computations in accordance with a single-instruction-multiple-data ("SIMD") paradigm. Thus, although various functionality is described herein as being performed by or in conjunction with the APD 316, in various alternatives, the functionality described as being performed by the APD 316 is additionally, or alternatively, performed by other computing devices having similar capabilities that are not driven by a host processor (e.g., processor 302) and provides graphical output to a display device 318. For example, it is contemplated that any processing system that performs processing tasks in accordance with a SIMD paradigm may perform the functionality described herein. Alternatively, it is contemplated that computing systems that do not perform processing tasks in accordance with a SIMD paradigm performs the functionality described herein.

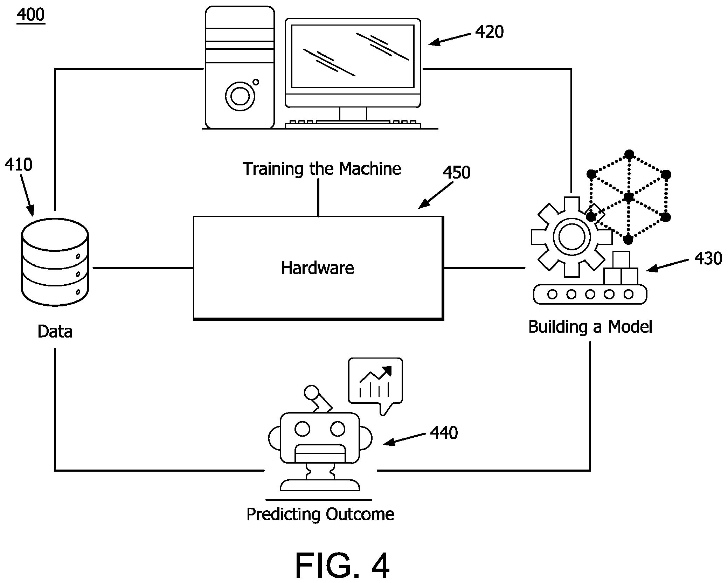

FIG. 4 illustrates a graphical depiction of an artificial intelligence system 200 incorporating the example device of FIG. 3. System 400 includes data 410, a machine 420, a model 430, a plurality of outcomes 440 and underlying hardware 450. System 400 operates by using the data 410 to train the machine 420 while building a model 430 to enable a plurality of outcomes 440 to be predicted. The system 400 may operate with respect to hardware 450. In such a configuration, the data 410 may be related to hardware 450 and may originate with apparatus 102, for example. For example, the data 410 may be on-going data, or output data associated with hardware 450. The machine 420 may operate as the controller or data collection associated with the hardware 450, or be associated therewith. The model 430 may be configured to model the operation of hardware 450 and model the data 410 collected from hardware 450 in order to predict the outcome achieved by hardware 450. Using the outcome 440 that is predicted, hardware 450 may be configured to provide a certain desired outcome 440 from hardware 450.

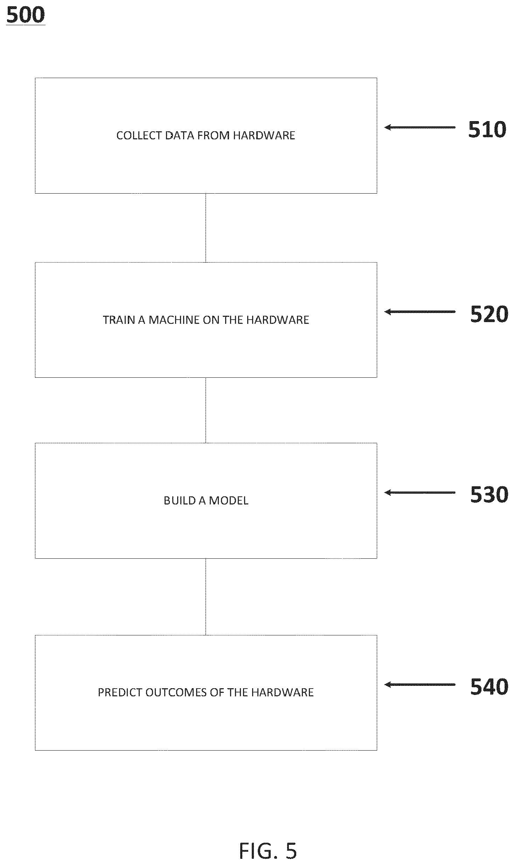

FIG. 5 illustrates a method 500 performed in the artificial intelligence system of FIG. 4. Method 500 includes collecting data from the hardware at step 510. This data may include currently collected, historical or other data from the hardware. For example, this data may include measurements during a surgical procedure and may be associated with the outcome of the procedure. For example, the temperature of a heart may be collected and correlated with the outcome of a heart procedure.

At step 520, method 500 includes training a machine on the hardware. The training may include an analysis and correlation of the data collected in step 510. For example, in the case of the heart, the data of temperature and outcome may be trained to determine if a correlation or link exists between the temperature of the heart during the procedure and the outcome.

At step 530, method 500 includes building a model on the data associated with the hardware. Building a model may include physical hardware or software modeling, algorithmic modeling and the like, as will be described below. This modeling may seek to represent the data that has been collected and trained.

At step 540, method 500 includes predicting the outcomes of the model associated with the hardware. This prediction of the outcome may be based on the trained model. For example, in the case of the heart, if the temperature during the procedure between 97.7 - 100.2 produces a positive result from the procedure, the outcome can be predicted in a given procedure based on the temperature of the heart during the procedure. While this model is rudimentary, it is provided for exemplary purposes and to increase understanding of the present teachings.

The present system and method operate to train the machine, build the model and predict outcomes using algorithms. These algorithms may be used to solve the trained model and predict outcomes associated with the hardware. These algorithms may be divided generally into classification, regression and clustering algorithms.

For example, a classification algorithm is used in the situation where the dependent variable, which is the variable being predicted, is divided into classes and predicting a class, the dependent variable, for a given input. Thus, a classification algorithm is used to predict an outcome, from a set number of fixed, predefined outcomes. A classification algorithm may include naive Bayes algorithms, decision trees, random forest classifiers, logistic regressions, support vector machines and k nearest neighbors.

Generally, a naive Bayes algorithm follows the Bayes theorem, and follows a probabilistic approach. As would be understood, other probabilistic-based algorithms may also be used, and generally operate using similar probabilistic principles to those described below for the exemplary naive Bayes algorithm.

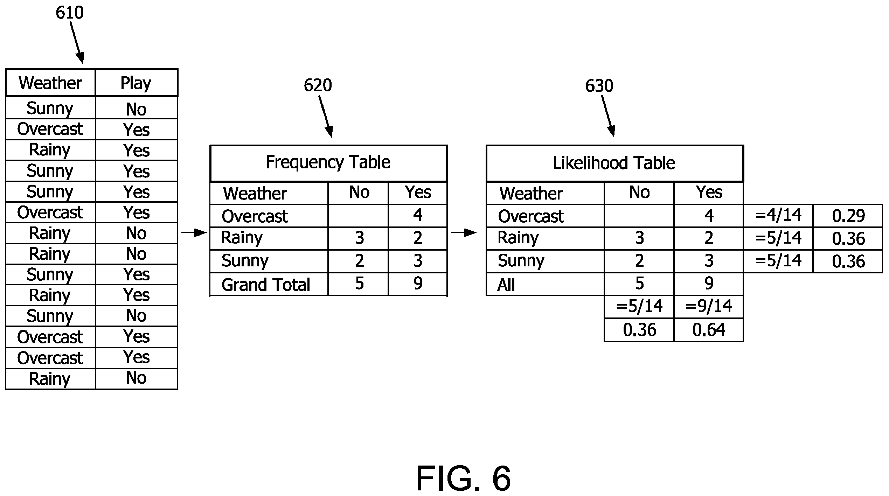

FIG. 6 illustrates an example of the probabilities of a naive Bayes calculation. The probability approach of Bayes theorem essentially means, that instead of jumping straight into the data, the algorithm has a set of prior probabilities for each of the classes for the target. After the data is entered, the naive Bayes algorithm may update the prior probabilities to form a posterior probability. This is given by the formula:

$$posterior = \frac{prior \ x \ likelihood}{evidence}$$

This naive Bayes algorithm, and Bayes algorithms generally, may be useful when needing to predict whether your input belongs to a given list of n classes or not. The probabilistic approach may be used because the probabilities for all the n classes will be quite low.

For example, as illustrated in FIG. 6, a person playing golf, which depends on factors including the weather outside shown in a first data set 610. The first data set 610 illustrates the weather in a first column and an outcome of playing associated with that weather in a second column. In the frequency table 620 the frequencies with which certain events occur are generated. In frequency table 620, the frequency of a person playing or not playing golf in each of the weather conditions is determined. From there, a likelihood table is compiled to generate initial probabilities. For example, the probability of the weather being overcast is 0.29 while the general probability of playing is 0.64.

The posterior probabilities may be generated from the likelihood table 630. These posterior probabilities may be configured to answer questions about weather conditions and whether golf is played in those weather conditions. For example, the probability of it being sunny outside and golf being played may be set forth by the Bayesian formula:

$$P(\text{Yes} \mid \text{Sunny}) = P(\text{Sunny} \mid \text{Yes}) * P(\text{Yes}) / P(\text{Sunny})$$

According to likelihood table 630:

$$P(\text{Sunny} \mid \text{Yes}) = 3/9 = 0.33,$$

$$P(\text{Sunny}) = 5/14 = 0.36,$$

$$P(\text{Yes}) = 9/14 = 0.64.$$

Therefore, the P(Yes | Sunny) = .33*.64/.36 or approximately 0.60 (60%).

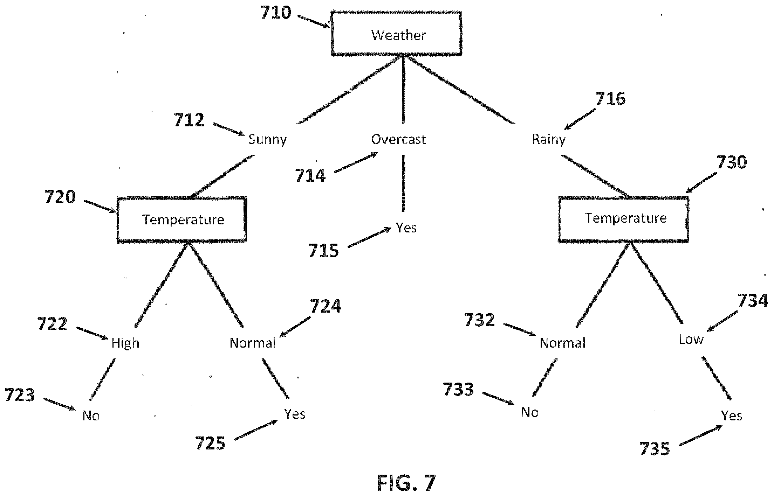

Generally, a decision tree is a flowchart-like tree structure where each external node denotes a test on an attribute and each branch represents the outcome of that test. The leaf nodes contain the actual predicted labels. The decision tree begins from the root of the tree with attribute values being compared until a leaf node is reached. A decision tree can be used as a classifier when handling high dimensional data and when little time has been spent behind data preparation. Decision trees may take the form of a simple decision tree, a linear decision tree, an algebraic decision tree, a deterministic decision tree, a randomized decision tree, a nondeterministic decision tree, and a quantum decision tree. An exemplary decision tree is provided below in FIG. 7.

FIG. 7 illustrates a decision tree, along the same structure as the Bayes example above, in deciding whether to play golf. In the decision tree, the first node 710 examines the weather providing sunny 712, overcast 714, and rain 716 as the choices to progress down the decision tree. If the weather is sunny, the leg of the tree is followed to a second node 720 examining the temperature. The temperature at node 720 may be high 722 or normal 724, in this example. If the temperature at node 720 is high 722, then the predicted outcome of "No" 723 golf occurs. If the temperature at node 720 is normal 724, then the predicted outcome of "Yes" 725 golf occurs.

Further, from the first node 710, an outcome overcast 714, "Yes" 715 golf occurs.

From the first node weather 710, an outcome of rain 716 results in the third node 730 (again) examining temperature. If the temperature at third node 730 is normal 732, then "Yes" 733 golf is played. If the temperature at third node 730 is low 734, then "No" 735 golf is played.

From this decision tree, a golfer plays golf if the weather is overcast 715, in normal temperature sunny weather 725, and in normal temperature rainy weather 733, while the golfer does not play if there are sunny high temperatures 723 or low rainy temperatures 735.

A random forest classifier is a committee of decision trees, where each decision tree has been fed a subset of the attributes of data and predicts on the basis of that subset. The mode of the actual predicted values of the decision trees are considered to provide an ultimate random forest answer. The random forest classifier, generally, alleviates overfitting, which is present in a standalone decision tree, leading to a much more robust and accurate classifier.

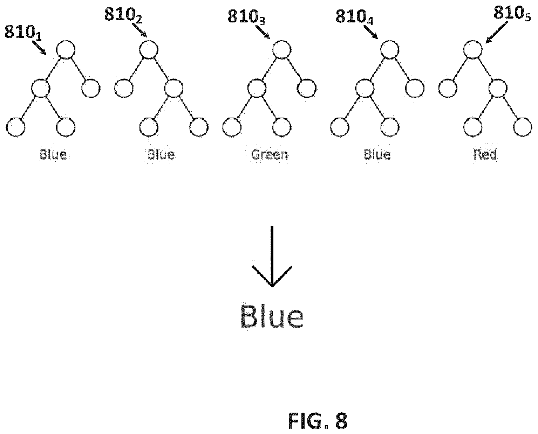

FIG. 8 illustrates an exemplary random forest classifier for classifying the color of a garment. As illustrated in FIG. 8, the random forest classifier includes five decision trees 8101, 8102, 8103, 8104, and 8105 (collectively or generally referred to as decision trees 810). Each of the trees is designed to classify the color of the garment. A discussion of each of the trees and decisions made is not provided, as each individual tree generally operates as the decision tree of FIG. 7. In the illustration, three (8101, 8102, 8104) of the five trees determines that the garment is blue, while one determines the garment is green (8103) and the remaining tree determines the garment is red (8105). The random forest takes these actual predicted values of the five trees and calculates the mode of the actual predicted values to provide random forest answer that the garment is blue.

Logistic Regression is another algorithm for binary classification tasks. Logistic regression is based on the logistic function, also called the sigmoid function. This S-shaped curve can take any real-valued number and map it between 0 and 1 asymptotically approaching those limits. The logistic model may be used to model the probability of a certain class or event existing such as pass/fail, win/lose, alive/dead or healthy/sick. This can be extended to model several classes of events such as determining whether an image contains a cat, dog, lion, etc. Each object being detected in the image would be assigned a probability between 0 and 1 with the sum of the probabilities adding to one.

In the logistic model, the log-odds (the logarithm of the odds) for the value labeled "1" is a linear combination of one or more independent variables ("predictors"); the independent variables can each be a binary variable (two classes, coded by an indicator variable) or a continuous variable (any real value). The corresponding probability of the value labeled "1" can vary between 0 (certainly the value "0") and 1 (certainly the value "1"), hence the labeling; the function that converts log-odds to probability is the logistic function, hence the name. The unit of measurement for the log-odds scale is called a logit, from logistic unit, hence the alternative names. Analogous models with a different sigmoid function instead of the logistic function can also be used, such as the probit model; the defining characteristic of the logistic model is that increasing one of the independent variables multiplicatively scales the odds of the given outcome at a constant rate, with each independent variable having its own parameter; for a binary dependent variable this generalizes the odds ratio.

In a binary logistic regression model, the dependent variable has two levels (categorical). Outputs with more than two values are modeled by multinomial logistic regression and, if the multiple categories are ordered, by ordinal logistic regression (for example the proportional odds ordinal logistic model). The logistic regression model itself simply models probability of output in terms of input and does not perform statistical classification (it is not a classifier), though it can be used to make a classifier, for instance by choosing a cutoff value and classifying inputs with probability greater than the cutoff as one class, below the cutoff as the other; this is a common way to make a binary classifier.

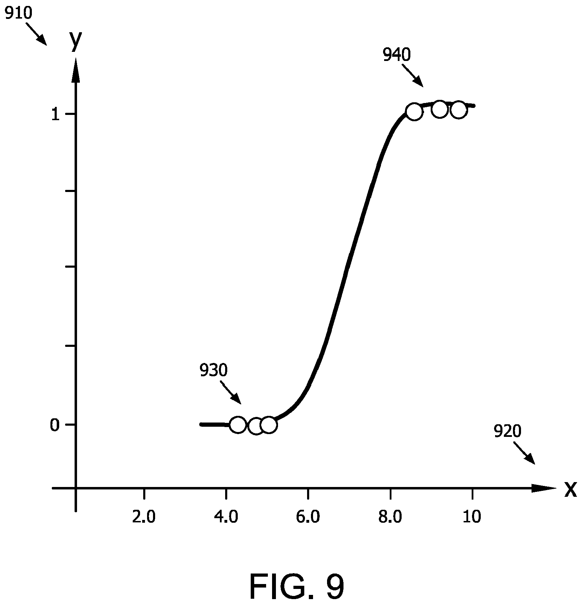

FIG. 9 illustrates an exemplary logistic regression. This exemplary logistic regression enables the prediction of an outcome based on a set of variables. For example, based on a person's grade point average, and outcome of being accepted to a school may be predicted. The past history of grade point averages and the relationship with acceptance enables the prediction to occur. The logistic regression of FIG. 9 enables the analysis of the grade point average variable 920 to predict the outcome 910 defined by 0 to 1. At the low end 930 of the S-shaped curve, the grade point average 920 predicts an outcome 910 of not being accepted. While at the high end 940 of the S-shaped curve, the grade point average 920 predicts an outcome 910 of being accepted. Logistic regression may be used to predict house values, customer lifetime value in the insurance sector, etc.

A support vector machine (SVM) may be used to sort the data with the margins between two classes as far apart as possible. This is called maximum margin separation. The SVM may account for the support vectors while plotting the hyperplane, unlike linear regression which uses the dataset for that purpose.

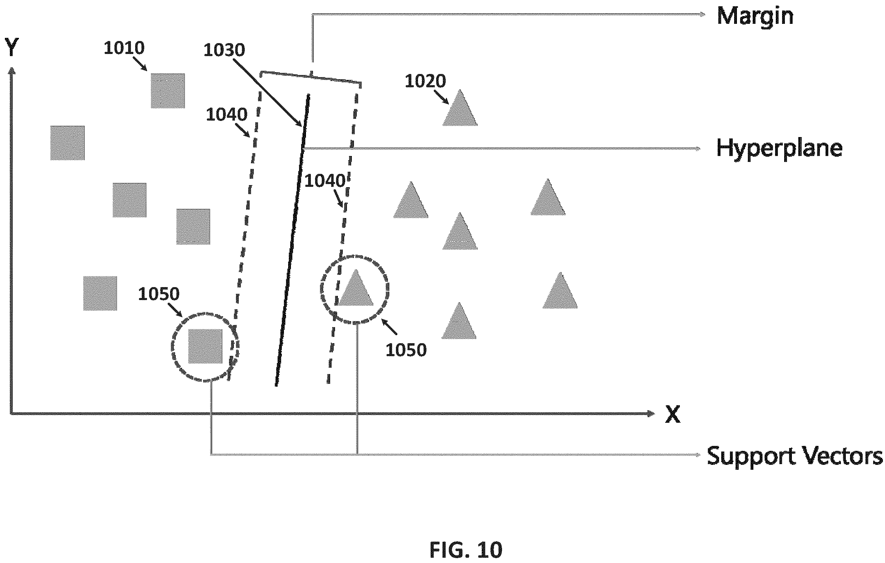

FIG. 10 illustrates an exemplary support vector machine. In the exemplary SVM 1000, data may be classified into two different classes represented as squares 1010 and triangles 1020. SVM 1000 operates by drawing a random hyperplane 1030. This hyperplane 1030 is monitored by comparing the distance (illustrated with lines 1040) between the hyperplane 1030 and the closest data points 1050 from each class. The closest data points 1050 to the hyperplane 1030 are known as support vectors. The hyperplane 1030 is drawn based on these support vectors 1050 and an optimum hyperplane has a maximum distance from each of the support vectors 1050. The distance between the hyperplane 1030 and the support vectors 1050 is known as the margin.

SVM 1000 may be used to classify data by using a hyperplane 1030, such that the distance between the hyperplane 1030 and the support vectors 1050 is maximum. Such an SVM 1000 may be used to predict heart disease, for example.

K Nearest Neighbors (KNN) refers to a set of algorithms that generally do not make assumptions on the underlying data distribution, and perform a reasonably short training phase. Generally, KNN uses many data points separated into several classes to predict the classification of a new sample point. Operationally, KNN specifies an integer N with a new sample. The N entries in the model of the system closest to the new sample are selected. The most common classification of these entries is determined, and that classification is assigned to the new sample. KNN generally requires the storage space to increase as the training set increases. This also means that the estimation time increases in proportion to the number of training points.

In regression algorithms, the output is a continuous quantity so regression algorithms may be used in cases where the target variable is a continuous variable. Linear regression is a general example of regression algorithms. Linear regression may be used to gauge genuine qualities (cost of houses, number of calls, all out deals and so forth) in view of the consistent variable(s). A connection between the variables and the outcome is created by fitting the best line (hence linear regression). This best fit line is known as regression line and spoken to by a direct condition Y= a *X + b. Linear regression is best used in approaches involving a low number of dimensions.

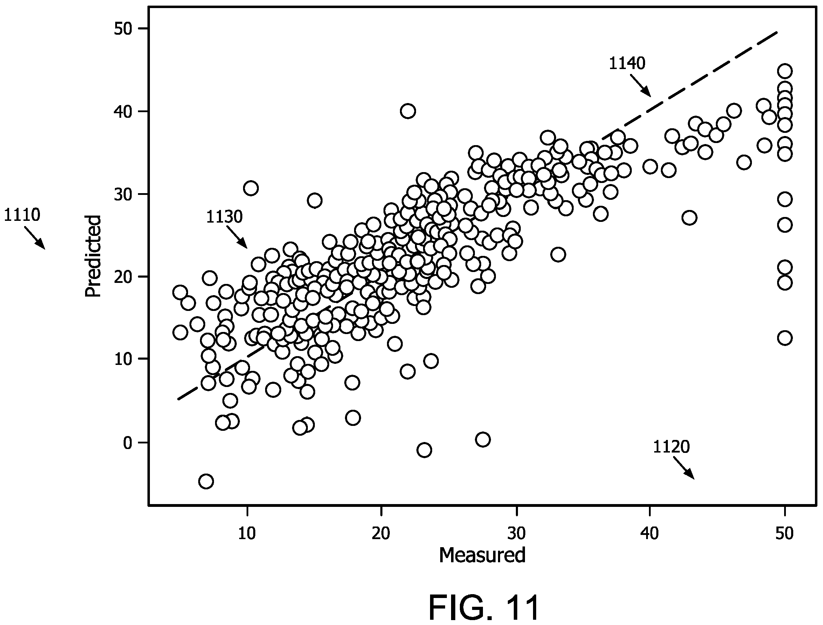

FIG. 11 illustrates an exemplary linear regression model. In this model, a predicted variable 1110 is modeled against a measured variable 1120. A cluster of instances of the predicted variable 1110 and measured variable 1120 are plotted as data points 1130. Data points 1130 are then fit with the best fit line 1140. Then the best fit line 1140 is used in subsequent predicted, given a measured variable 1120, the line 1140 is used to predict the predicted variable 1110 for that instance. Linear regression may be used to model and predict in a financial portfolio, salary forecasting, real estate and in traffic in arriving at estimated time of arrival.

Clustering algorithms may also be used to model and train on a data set. In clustering, the input is assigned into two or more clusters based on feature similarity. Clustering algorithms generally learn the patterns and useful insights from data without any guidance. For example, clustering viewers into similar groups based on their interests, age, geography, etc. may be performed using unsupervised learning algorithms like K-means clustering.

K-means clustering generally is regarded as a simple unsupervised learning approach. In K-means clustering similar data points may be gathered together and bound in the form of a cluster. One method for binding the data points together is by calculating the centroid of the group of data points. In determining effective clusters, in K-means clustering the distance between each point from the centroid of the cluster is evaluated. Depending on the distance between the data point and the centroid, the data is assigned to the closest cluster. The goal of clustering is to determine the intrinsic grouping in a set of unlabeled data. The 'K' in K-means stands for the number of clusters formed. The number of clusters (basically the number of classes in which new instances of data may be classified) may be determined by the user. This determination may be performed using feedback and viewing the size of the clusters during training, for example.

K-means is used majorly in cases where the data set has points which are distinct and well separated, otherwise, if the clusters are not separated the modeling may render the clusters inaccurate. Also, K-means may be avoided in cases where the data set contains a high number of outliers or the data set is non-linear.

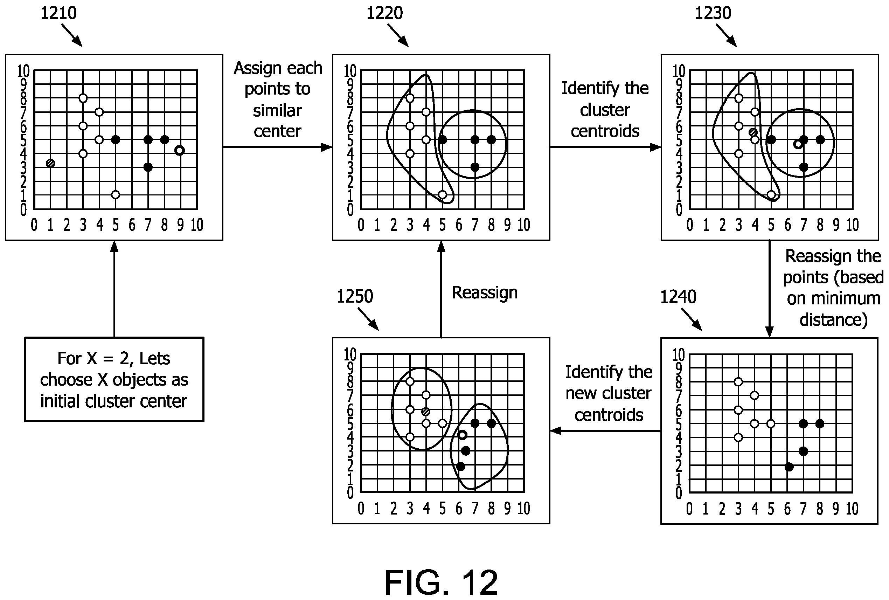

FIG. 12 illustrates a K-means clustering. In K-means clustering, the data points are plotted, and the K value is assigned. For example, for K=2 in FIG. 12, the data points are plotted as shown in depiction 1210. The points are then assigned to similar centers at step 1220. The cluster centroids are identified as shown in 1230. Once centroids are identified, the points are reassigned to the cluster to provide the minimum distance between the data point to the respective cluster centroid as illustrated in 1240. Then a new centroid of the cluster may be determined as illustrated in depiction 1250. As the data points are reassigned to a cluster, new cluster centroids formed, an iteration, or series of iterations, may occur to enable the clusters to be minimized in size and the centroid of the optimal centroid determined. Then as new data points are measured, the new data points may be compared with the centroid and cluster to identify with that cluster.

Ensemble learning algorithms may be used. These algorithms use multiple learning algorithms to obtain better predictive performance than could be obtained from any of the constituent learning algorithms alone. Ensemble learning algorithms perform the task of searching through a hypothesis space to find a suitable hypothesis that will make good predictions with a particular problem. Even if the hypothesis space contains hypotheses that are very well-suited for a particular problem, it may be very difficult to find a good hypothesis. Ensemble algorithms combine multiple hypotheses to form a better hypothesis. The term ensemble is usually reserved for methods that generate multiple hypotheses using the same base learner. The broader term of multiple classifier systems also covers hybridization of hypotheses that are not induced by the same base learner.

Evaluating the prediction of an ensemble typically requires more computation than evaluating the prediction of a single model, so ensembles may be thought of as a way to compensate for poor learning algorithms by performing a lot of extra computation. Fast algorithms such as decision trees are commonly used in ensemble methods, for example, random forests, although slower algorithms can benefit from ensemble techniques as well.

An ensemble is itself a supervised learning algorithm, because it can be trained and then used to make predictions. The trained ensemble, therefore, represents a single hypothesis. This hypothesis, however, is not necessarily contained within the hypothesis space of the models from which it is built. Thus, ensembles can be shown to have more flexibility in the functions they can represent. This flexibility can, in theory, enable them to over-fit the training data more than a single model would, but in practice, some ensemble techniques (especially bagging) tend to reduce problems related to over-fitting of the training data.

Empirically, ensemble algorithms tend to yield better results when there is a significant diversity among the models. Many ensemble methods, therefore, seek to promote diversity among the models they combine. Although non-intuitive, more random algorithms (like random decision trees) can be used to produce a stronger ensemble than very deliberate algorithms (like entropy-reducing decision trees). Using a variety of strong learning algorithms, however, has been shown to be more effective than using techniques that attempt to dumb-down the models in order to promote diversity.

The number of component classifiers of an ensemble has a great impact on the accuracy of prediction. A priori determining of ensemble size and the volume and velocity of big data streams make this even more crucial for online ensemble classifiers. A theoretical framework suggests that there are an ideal number of component classifiers for an ensemble such that having more or less than this number of classifiers would deteriorate the accuracy. The theoretical framework shows that using the same number of independent component classifiers as class labels gives the highest accuracy.

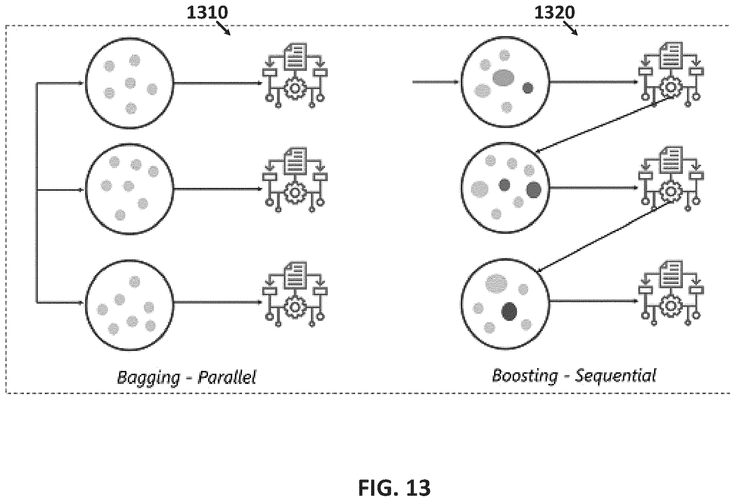

Some common types of ensembles include Bayes optimal classifier, bootstrap aggregating (bagging), boosting, Bayesian model averaging, Bayesian model combination, bucket of models and stacking. FIG. 13 illustrates an exemplary ensemble learning algorithm where bagging is being performed in parallel 1310 and boosting is being performed sequentially 1320.

A neural network is a network or circuit of neurons, or in a modern sense, an artificial neural network, composed of artificial neurons or nodes. The connections of the biological neuron are modeled as weights. A positive weight reflects an excitatory connection, while negative values mean inhibitory connections. Inputs are modified by a weight and summed using a linear combination. An activation function may control the amplitude of the output. For example, an acceptable range of output is usually between 0 and 1, or it could be -1 and 1.

These artificial networks may be used for predictive modeling, adaptive control and applications and can be trained via a dataset. Self-learning resulting from experience can occur within networks, which can derive conclusions from a complex and seemingly unrelated set of information.

For completeness, a biological neural network is composed of a group or groups of chemically connected or functionally associated neurons. A single neuron may be connected to many other neurons and the total number of neurons and connections in a network may be extensive. Connections, called synapses, are usually formed from axons to dendrites, though dendrodendritic synapses and other connections are possible. Apart from the electrical signaling, there are other forms of signaling that arise from neurotransmitter diffusion.

Artificial intelligence, cognitive modeling, and neural networks are information processing paradigms inspired by the way biological neural systems process data. Artificial intelligence and cognitive modeling try to simulate some properties of biological neural networks. In the artificial intelligence field, artificial neural networks have been applied successfully to speech recognition, image analysis and adaptive control, in order to construct software agents (in computer and video games) or autonomous robots.

A neural network (NN), in the case of artificial neurons called artificial neural network (ANN) or simulated neural network (SNN), is an interconnected group of natural or artificial neurons that uses a mathematical or computational model for information processing based on a connectionistic approach to computation. In most cases an ANN is an adaptive system that changes its structure based on external or internal information that flows through the network. In more practical terms neural networks are non-linear statistical data modeling or decision-making tools. They can be used to model complex relationships between inputs and outputs or to find patterns in data.

An artificial neural network involves a network of simple processing elements (artificial neurons) which can exhibit complex global behavior, determined by the connections between the processing elements and element parameters.

One classical type of artificial neural network is the recurrent Hopfield network. The utility of artificial neural network models lies in the fact that they can be used to infer a function from observations and also to use it. Unsupervised neural networks can also be used to learn representations of the input that capture the salient characteristics of the input distribution, and more recently, deep learning algorithms, which can implicitly learn the distribution function of the observed data. Learning in neural networks is particularly useful in applications where the complexity of the data or task makes the design of such functions by hand impractical.

Neural networks can be used in different fields. The tasks to which artificial neural networks are applied tend to fall within the following broad categories: function approximation, or regression analysis, including time series prediction and modeling; classification, including pattern and sequence recognition, novelty detection and sequential decision making, data processing, including filtering, clustering, blind signal separation and compression.

Application areas of ANNs include nonlinear system identification and control (vehicle control, process control), game-playing and decision making (backgammon, chess, racing), pattern recognition (radar systems, face identification, object recognition), sequence recognition (gesture, speech, handwritten text recognition), medical diagnosis, financial applications, data mining (or knowledge discovery in databases, "KDD"), visualization and e-mail spam filtering. For example, it is possible to create a semantic profile of user's interests emerging from pictures trained for object recognition.

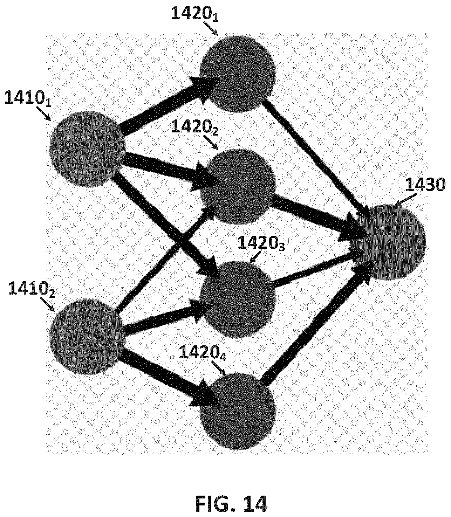

FIG. 14 illustrates an exemplary neural network. In the neural network there is an input layer represented by a plurality of inputs, such as $1410_1$ and $1410_2$. The inputs $1410_1$, $1410_2$ are provided to a hidden layer depicted as including nodes $1420_1$, $1420_2$, $1420_3$, $1420_4$. These nodes $1420_1$, $1420_2$, $1420_3$, $1420_4$ are combined to produce an output 1430 in an output layer. The neural network performs simple processing via the hidden layer of simple processing elements, nodes $1420_1$, $1420_2$, $1420_3$, $1420_4$, which can exhibit complex global behavior, determined by the connections between the processing elements and element parameters.

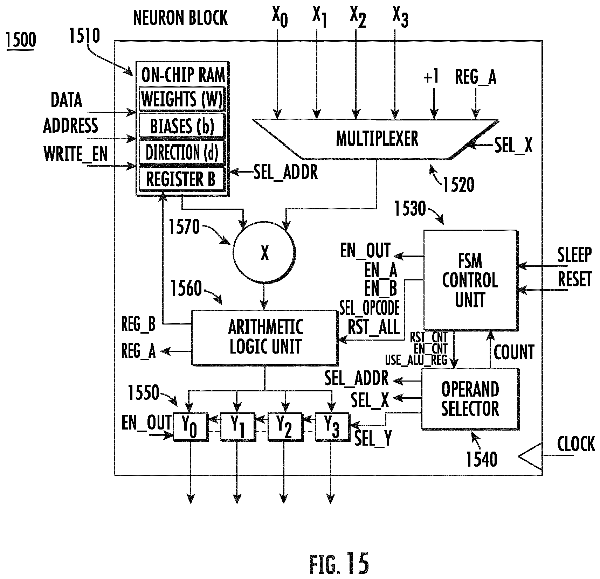

The neural network of FIG. 14 may be implemented in hardware. As depicted in FIG. 15 a hardware based neural network is depicted.

Cardiac arrhythmias, and atrial fibrillation in particular, persist as common and dangerous medical ailments, especially in the aging population. In patients with normal sinus rhythm, the heart, which is comprised of atrial, ventricular, and excitatory conduction tissue, is electrically excited to beat in a synchronous, patterned fashion. In patients with cardiac arrythmias, abnormal regions of cardiac tissue do not follow the synchronous beating cycle associated with normally conductive tissue as in patients with normal sinus rhythm. Instead, the abnormal regions of cardiac tissue aberrantly conduct to adjacent tissue, thereby disrupting the cardiac cycle into an asynchronous cardiac rhythm. Such abnormal conduction has been previously known to occur at various regions of the heart, for example, in the region of the sino-atrial (SA) node, along the conduction pathways of the atrioventricular (AV) node and the Bundle of His, or in the cardiac muscle tissue forming the walls of the ventricular and atrial cardiac chambers.

Cardiac arrhythmias, including atrial arrhythmias, may be of a multiwavelet reentrant type, characterized by multiple asynchronous loops of electrical impulses that are scattered about the atrial chamber and are often selfpropagating. Alternatively, or in addition to the multiwavelet reentrant type, cardiac arrhythmias may also have a focal origin, such as when an isolated region of tissue in an atrium fires autonomously in a rapid, repetitive fashion. Ventricular tachycardia (V-tach or VT) is a tachycardia, or fast heart rhythm that originates in one of the ventricles of the heart. This is a potentially life-threatening arrhythmia because it may lead to ventricular fibrillation and sudden death.

One type of arrhythmia, atrial fibrillation, occurs when the normal electrical impulses generated by the sinoatrial node are overwhelmed by disorganized electrical impulses that originate in the atria and pulmonary veins causing irregular impulses to be conducted to the ventricles. An irregular heartbeat results and may last from minutes to weeks, or even years. Atrial fibrillation (AF) is often a chronic condition that leads to a small increase in the risk of death often due to strokes. Risk increases with age. Approximately 8% of people over 80 having some amount of AF. Atrial fibrillation is often asymptomatic and is not in itself generally life-threatening, but it may result in palpitations, weakness, fainting, chest pain and congestive heart failure. Stroke risk increases during AF because blood may pool and form clots in the poorly contracting atria and the left atrial appendage. The first line of treatment for AF is medication that either slow the heart rate or revert the heart rhythm back to normal. Additionally, persons with AF are often given anticoagulants to protect them from the risk of stroke. The use of such anticoagulants comes with its own risk of internal bleeding. In some patients, medication is not sufficient, and their AF is deemed to be drug-refractory, i.e., untreatable with standard pharmacological interventions. Synchronized electrical cardioversion may also be used to convert AF to a normal heart rhythm. Alternatively, AF patients are treated by catheter ablation.

A catheter ablation-based treatment may include mapping the electrical properties of heart tissue, especially the endocardium and the heart volume, and selectively ablating cardiac tissue by application of energy. Cardiac mapping, for example, creating a map of electrical potentials (a voltage map) of the wave propagation along the heart tissue or a map of arrival times (a local time activation (LAT) map) to various tissue located points, may be used for detecting local heart tissue dysfunction Ablations, such as those based on cardiac mapping, can cease or modify the propagation of unwanted electrical signals from one portion of the heart to another.

The ablation process damages the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure-mapping followed by ablation-electrical activity at points within the heart is typically sensed and measured by advancing a catheter containing one or more electrical sensors (or electrodes) into the heart, and acquiring data at a multiplicity of points. These data are then utilized to select the endocardial target areas at which ablation is to be performed.

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems in order to reconstruct the anatomy of the heart chamber of interest.

For example, cardiologists rely upon software such as the Complex Fractionated Atrial Electrograms (CFAE) module of the CARTO®3 3D mapping system, produced by Biosense Webster, Inc. (Diamond Bar, Calif.), to analyze intracardiac EGM signals and determine the ablation points for treatment of a broad range of cardiac conditions, including atypical atrial flutter and ventricular tachycardia.

The 3D maps can provide multiple pieces of information regarding the electrophysiological properties of the tissue that represent the anatomical and functional substrate of these challenging arrhythmias.

Cardiomyopathies with different etiologies (ischemic, dilated cardiomyopathy (DCM), hypertrophic cardiomyopathy (HCM), arrhythmogenic right ventricular dysplasia (ARVD), left ventricular non-compaction (LVNC), etc.) have an identifiable substrate, featured by areas of unhealthy tissue surrounded by areas of normally functioning cardiomyocytes.

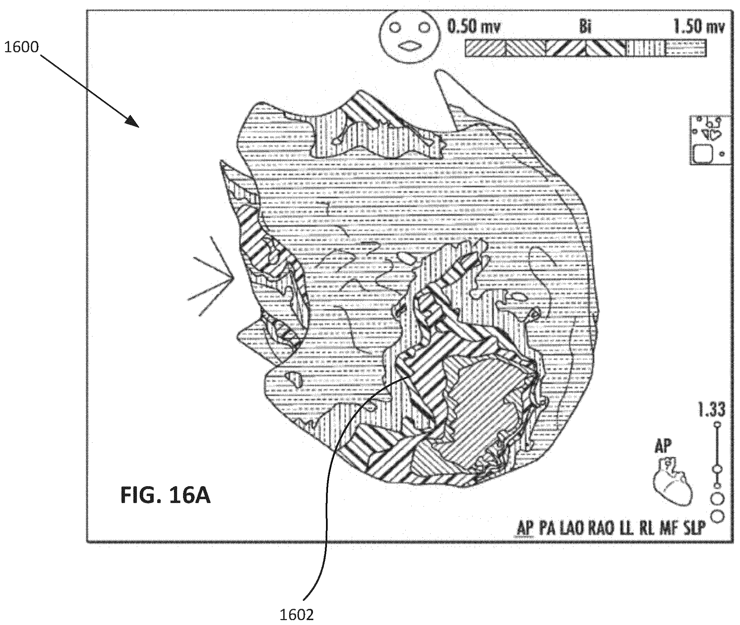

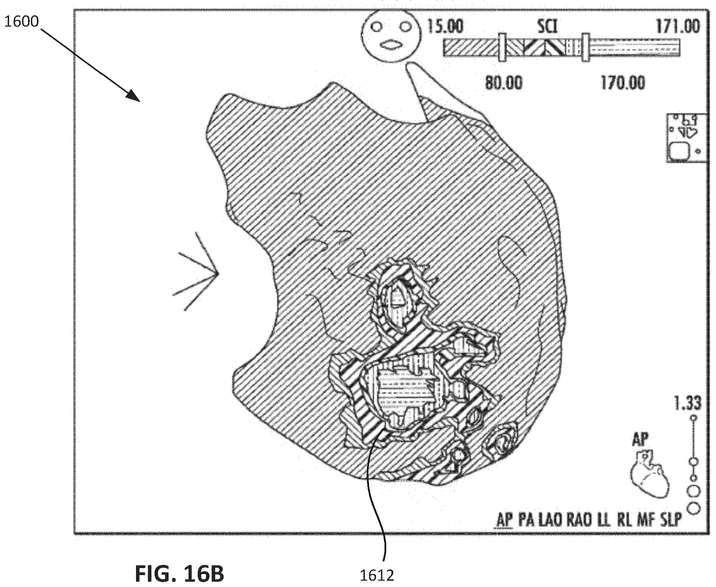

FIGS. 16A through 16D show examples of cardiomyopathies with different etiologies. As a first example, FIGs. 16A and 16B show an example rendering of a heart 1600 with post-ischemic Ventricular Tachycardia (VT) characterized by endo-epicardial low or intermediate voltage area 1602 in which signal conduction is slowed down. This illustrates that measuring any prolonged potential inside or around the dense scar area may help identify potential isthmuses sustaining VT. The post-ischemic VT shown in FIG. 16A is characterized by an endo-epicardial low or intermediate voltage area in which signal conduction is slowed down. This illustrates that measuring any prolonged potential inside or around the dense scar area may help identify potential isthmuses sustaining VT. FIG. 16A illustrates the bipolar signal amplitude (Bi) variance in the various sectors of the heart 1600. FIG. 16A shows Bi ranges from 0.5 mV to 1.5 mV. FIG. 16B illustrates the Shortex Complex Interval (SCI) variance in the various sectors of the heart. As an example, SCI ranges from 15.0 msec to 171.00 msec with the SCI range of interest between 80 msec and 170 msec.

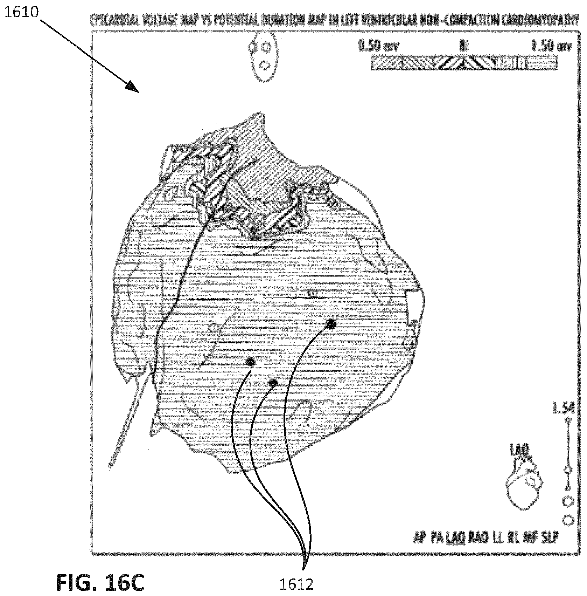

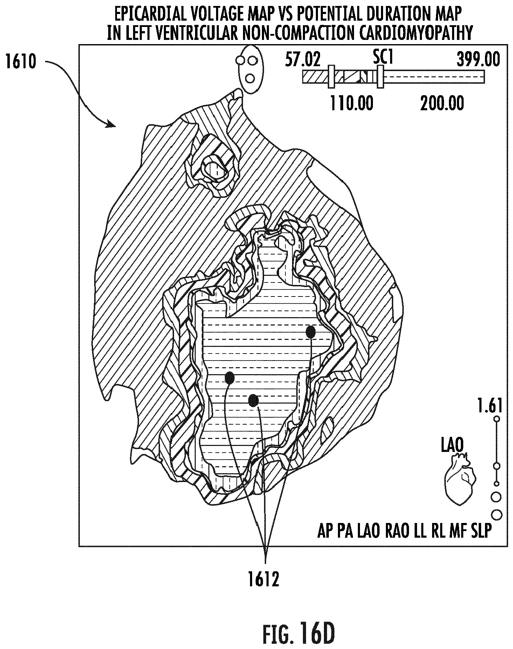

FIGs. 16C and 16D show an example rendering of a heart 1610 experiencing left ventricular non-compaction cardiomyopathy. More specifically, FIG. 16C shows an epicardial voltage map and FIG. 16D shows potential duration map (PDM). The three black circles in 1612 in FIGs. 16C and 16D are marked as abnormally prolonged potentials, e.g., potentials above 200 msec.

Abnormal tissue is generally characterized by low-voltage EGMs. However, initial clinical experience in endo-epicardial mapping indicates that areas of low-voltage are not always present as the sole arrhythmogenic mechanism in such patients. In fact, areas of low or medium voltage may exhibit EGM fragmentation and prolonged activities during sinus rhythm, which corresponds to the critical isthmus identified during sustained and organized ventricular arrhythmias, e.g., applies to non-tolerated ventricular tachycardias. Moreover, in many cases, EGM fragmentation and prolonged activities are observed in the regions showing a normal or near-normal voltage amplitude (> 1-1.5 mV). Although the latter areas may be evaluated according to the voltage amplitude, they cannot be considered as normal according to the intracardiac signal, thus representing a true arrhythmogenic substrate. The 3D mapping may be able to localize the arrhythmogenic substrate on the endocardial and/or epicardial layer of the right/left ventricle, which may vary in distribution according to the extension of the main disease.

The substrate linked to these cardiac conditions is related to the presence of fragmented and prolonged EGMs in the endocardial and/or epicardial layers of the ventricular chambers (right and left). The 3D mapping system, such as CARTO®3, is able to localize the potential arrhythmogenic substrate of the cardiomyopathy in terms of abnormal EGM detection.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral artery, and then guided into the chamber of the heart of concern. A typical ablation procedure involves the insertion of a catheter having at least one electrode at its distal end, into a heart chamber. A reference electrode is provided, generally taped to the skin of the patient or by means of a second catheter that is positioned in or near the heart. RF (radio frequency) current is applied to the tip electrode of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the electrode also occurs as a result of conduction from the heated tissue to the electrode itself. If the electrode temperature becomes sufficiently high, possibly above 60 degrees C., a thin transparent coating of dehydrated blood protein can form on the surface of the electrode. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs, and the catheter may be removed from the body and the tip electrode cleaned.

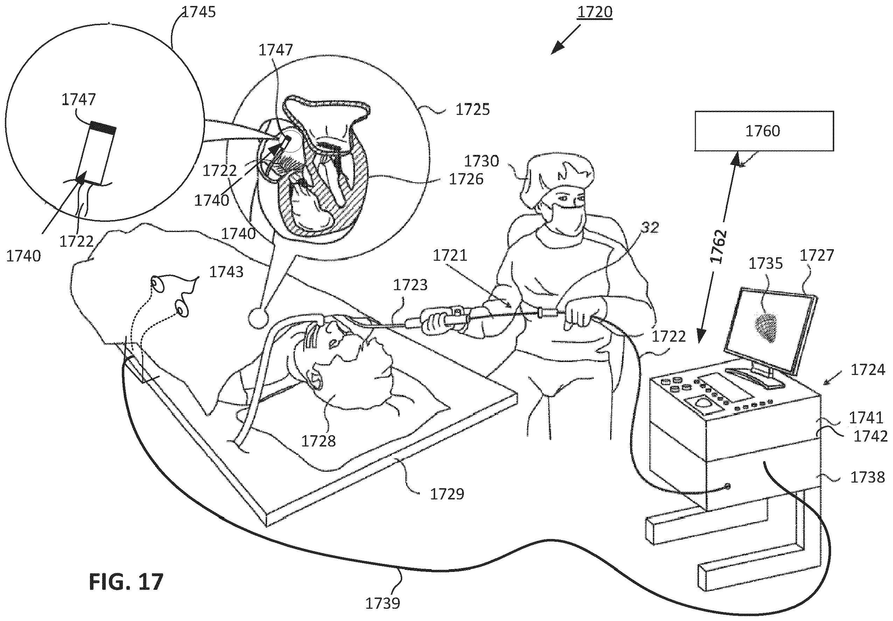

FIG. 17 is a diagram of an exemplary system 1720 in which one or more features of the disclosure subject matter can be implemented. All or parts of system 1720 may be used to collect information for a training dataset and/or all or parts of system 1720 may be used to implement a trained model. System 1720 may include components, such as a catheter 1740, that are configured to damage tissue areas of an intra-body organ. The catheter 1740 may also be further configured to obtain biometric data. Although catheter 1740 is shown to be a point catheter, it will be understood that a catheter of any shape that includes one or more elements (e.g., electrodes) may be used to implement the embodiments disclosed herein. System 1720 includes a probe 1721, having shafts that may be navigated by a physician 1730 into a body part, such as heart 1726, of a patient 1728 lying on a table 1729. According to embodiments, multiple probes may be provided, however, for purposes of conciseness, a single probe 1721 is described herein but it will be understood that probe 1721 may represent multiple probes. As shown in FIG. 17, physician 1730 may insert shaft 1722 through a sheath 1723, while manipulating the distal end of the shafts 1722 using a manipulator 1732 near the proximal end of the catheter 1740 and/or deflection from the sheath 1723. As shown in an inset 1725, catheter 1740 may be fitted at the distal end of shafts 1722. Catheter 1740 may be inserted through sheath 1723 in a collapsed state and may be then expanded within heart 1726. Cather 1740 may include at least one ablation electrode 1747 and a catheter needle 1748, as further disclosed herein.

According to exemplary embodiments, catheter 1740 may be configured to ablate tissue areas of a cardiac chamber of heart 1726. Inset 1745 shows catheter 1740 in an enlarged view, inside a cardiac chamber of heart 1726. As shown, catheter 1740 may include at least one ablation electrode 1747 coupled onto the body of the catheter. According to other exemplary embodiments, multiple elements may be connected via splines that form the shape of the catheter 1740. One or more other elements (not shown) may be provided and may be any elements configured to ablate or to obtain biometric data and may be electrodes, transducers, or one or more other elements.

According to embodiments disclosed herein, the ablation electrodes, such as electrode 1747, may be configured to provide energy to tissue areas of an intra-body organ such as heart 1726. The energy may be thermal energy and may cause damage to the tissue area starting from the surface of the tissue area and extending into the thickness of the tissue area.

According to exemplary embodiments disclosed herein, biometric data may include one or more of local activation times (LATs), electrical activity, topology, bipolar mapping, dominant frequency, impedance, or the like. The LAT may be a point in time of a threshold activity corresponding to a local activation, calculated based on a normalized initial starting point. Electrical activity may be any applicable electrical signals that may be measured based on one or more thresholds and may be sensed and/or augmented based on signal to noise ratios and/or other filters. A topology may correspond to the physical structure of a body part or a portion of a body part and may correspond to changes in the physical structure relative to different parts of the body part or relative to different body parts. A dominant frequency may be a frequency or a range of frequency that is prevalent at a portion of a body part and may be different in different portions of the same body part. For example, the dominant frequency of a pulmonary vein of a heart may be different than the dominant frequency of the right atrium of the same heart. Impedance may be the resistance measurement at a given area of a body part.

As shown in FIG. 17, the probe 1721, and catheter 1740 may be connected to a console 1724. Console 1724 may include a processor 1741, such as a general-purpose computer, with suitable front end and interface circuits 1738 for transmitting and receiving signals to and from catheter, as well as for controlling the other components of system 1720. In some embodiments, processor 1741 may be further configured to receive biometric data, such as electrical activity, and determine if a given tissue area conducts electricity. According to an embodiment, the processor may be external to the console 1724 and may be located, for example, in the catheter, in an external device, in a mobile device, in a cloud-based device, or may be a standalone processor.

As noted above, processor 1741 may include a general-purpose computer, which may be programmed in software to carry out the functions described herein. The software may be downloaded to the general-purpose computer in electronic form, over a network, for example, or it may, alternatively or additionally, be provided and/or stored on non-transitory tangible media, such as magnetic, optical, or electronic memory. The example configuration shown in FIG. 17 may be modified to implement the embodiments disclosed herein. The disclosed embodiments may similarly be applied using other system components and settings. Additionally, system 1620 may include additional components, such as elements for sensing electrical activity, wired or wireless connectors, processing and display devices, or the like.

According to an embodiment, a display connected to a processor (e.g., processor 1741) may be located at a remote location such as a separate hospital or in separate healthcare provider networks. Additionally, the system 1720 may be part of a surgical system that is configured to obtain anatomical and electrical measurements of a patient's organ, such as a heart, and performing a cardiac ablation procedure. An example of such a surgical system is the Carto® system sold by Biosense Webster.

The system 1720 may also, and optionally, obtain biometric data such as anatomical measurements of the patient's heart using ultrasound, computed tomography (CT), magnetic resonance imaging (MRI) or other medical imaging techniques known in the art. The system 1720 may obtain electrical measurements using catheters, electrocardiograms (EKGs) or other sensors that measure electrical properties of the heart. The biometric data including anatomical and electrical measurements may then be stored in a memory 1742 of the mapping system 1720, as shown in FIG. 17. The biometric data may be transmitted to the processor 1741 from the memory 1742. Alternatively, or in addition, the biometric data may be transmitted to a server 1760, which may be local or remote, using a network 1662.

Network 1762 may be any network or system generally known in the art such as an intranet, a local area network (LAN), a wide area network (WAN), a metropolitan area network (MAN), a direct connection or series of connections, a cellular telephone network, or any other network or medium capable of facilitating communication between the mapping system 1720 and the server 1760. The network 1662 may be wired, wireless or a combination thereof. Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 1762.

In some instances, the server 1762 may be implemented as a physical server. In other instances, server 1762 may be implemented as a virtual server a public cloud computing provider (e.g., Amazon Web Services (AWS) ®).

Control console 1724 may be connected, by a cable 1739, to body surface electrodes 1743, which may include adhesive skin patches that are affixed to the patient 1730. The processor, in conjunction with a current tracking module, may determine position coordinates of the catheter 1740 inside the body part (e.g., heart 1726) of a patient. The position coordinates may be based on impedances or electromagnetic fields measured between the body surface electrodes 1743 and the electrode 1748 or other electromagnetic components of the catheter 1740. Additionally, or alternatively, location pads may be located on the surface of bed 1729 and may be separate from the bed 1729.

Processor 1741 may include real-time noise reduction circuitry typically configured as a field programmable gate array (FPGA), followed by an analog-to-digital (A/D) ECG (electrocardiograph) or EMG (electromyogram) signal conversion integrated circuit. The processor 1741 may pass the signal from an A/D ECG or EMG circuit to another processor and/or can be programmed to perform one or more functions disclosed herein.

Control console 1724 may also include an input/output (I/O) communications interface that enables the control console to transfer signals from, and/or transfer signals to electrode 1747.

During a procedure, processor 1741 may facilitate the presentation of a body part rendering 1735 to physician 1730 on a display 1727, and store data representing the body part rendering 1735 in a memory 1742. Memory 1742 may comprise any suitable volatile and/or non-volatile memory, such as random-access memory or a hard disk drive. In some embodiments, medical professional 1730 may be able to manipulate a body part rendering 1735 using one or more input devices such as a touch pad, a mouse, a keyboard, a gesture recognition apparatus, or the like. For example, an input device may be used to change the position of catheter 1740 such that rendering 1735 is updated. In alternative embodiments, display 1727 may include a touchscreen that can be configured to accept inputs from medical professional 1730, in addition to presenting a body part rendering 1735.

Treatments for cardiac conditions such as cardiac arrhythmia often require obtaining a detailed mapping of cardiac tissue, chambers, veins, arteries and/or electrical pathways. For example, a prerequisite for performing a catheter ablation successfully is that the cause of the cardiac arrhythmia is accurately located in the heart chamber. Such locating may be done via an electrophysiological investigation during which electrical potentials are detected spatially resolved with a mapping catheter introduced into the heart chamber. This electrophysiological investigation, the so-called electro-anatomical mapping, thus provides 3D mapping data which can be displayed on a monitor. In many cases, the mapping function and a treatment function (e.g., ablation) are provided by a single catheter or group of catheters such that the mapping catheter also operates as a treatment (e.g., ablation) catheter at the same time

Mapping of cardiac areas such as cardiac regions, tissue, veins, arteries and/or electrical pathways of the heart may result in identifying problem areas such as scar tissue, arrythmia sources (e.g., electric rotors), healthy areas, and the like. Cardiac areas may be mapped such that a visual rendering of the mapped cardiac areas is provided using a display, as further disclosed herein. Additionally, cardiac mapping may include mapping based on one or more modalities such as, but not limited to, LAT, an electrical activity, a topology, a bipolar mapping, a dominant frequency, or an impedance. Data corresponding to multiple modalities may be captured using a catheter inserted into a patient's body and may provide for rendering at the same time or at different times based on corresponding settings and/or preferences of a medical professional.

Cardiac mapping may be implemented using one or more techniques. As an example of a first technique, cardiac mapping may be implemented by sensing an electrical property of heart tissue, for example, LAT, as a function of the precise location within the heart. The corresponding data may be acquired with one or more catheters that are advanced into the heart using catheters that have electrical and location sensors in their distal tips. As specific examples, location and electrical activity may be initially measured on about 10 to about 20 points on the interior surface of the heart. These data points may be generally sufficient to generate a preliminary reconstruction or map of the cardiac surface to a satisfactory quality. The preliminary map may be combined with data taken at additional points in order to generate a more comprehensive map of the heart's electrical activity. In clinical settings, it is not uncommon to accumulate data at 100 or more sites to generate a detailed, comprehensive map of heart chamber electrical activity. The generated detailed map may then serve as the basis for deciding on a therapeutic course of action, for example, tissue ablation, to alter the propagation of the heart's electrical activity and to restore normal heart rhythm.

Catheters containing position sensors may be used to determine the trajectory of points on the cardiac surface. These trajectories may be used to infer motion characteristics such as the contractility of the tissue. Maps depicting such motion characteristics may be constructed when the trajectory information is sampled at a sufficient number of points in the heart.

Electrical activity at a point in the heart may be typically measured by advancing a catheter containing an electrical sensor at or near its distal tip to that point in the heart, contacting the tissue with the sensor and acquiring data at that point. One drawback with mapping a cardiac chamber using a catheter containing a single, distal tip electrode is the long period of time required to accumulate data on a point-by-point basis over the requisite number of points required for a detailed map of the chamber as a whole. Accordingly, multiple-electrode catheters have been developed to simultaneously measure electrical activity at multiple points in the heart chamber.

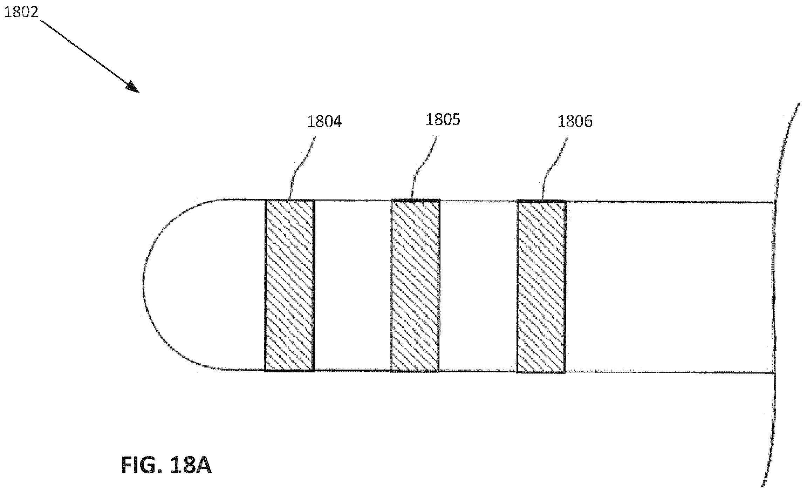

Multiple-electrode catheters may be implemented using any applicable shape such as a linear catheter with multiple electrodes, a balloon catheter including electrodes dispersed on multiple spines that shape the balloon, a lasso or loop catheter with multiple electrodes, or any other applicable shape. FIG. 18A shows an example of a linear catheter 1802 including multiple electrodes 1804, 1805, and 1806 that may be used to map a cardiac area. Linear catheter 1802 may be fully or partially elastic such that it can twist, bend, and or otherwise change its shape based on received signal and/or based on application of an external force (e.g., cardiac tissue) on the linear catheter 1802.

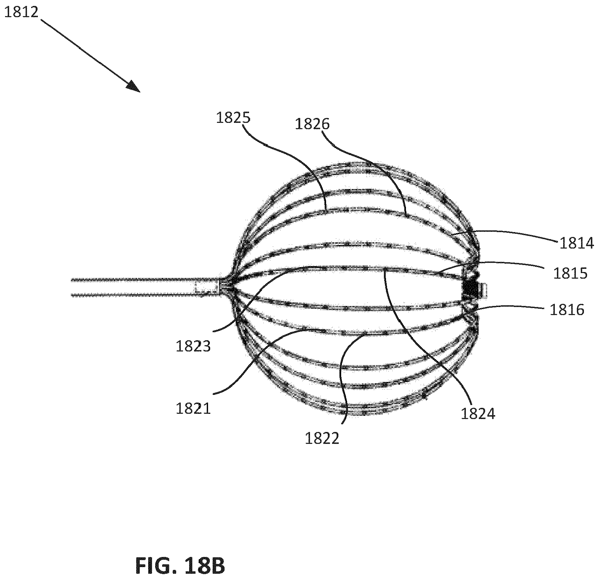

FIG. 18B shows an example of a balloon catheter 1812 including multiple splines. In the example depicted in FIG. 18B there are 12 splines including splines 1814, 1815, 1816 and multiple electrodes on each spline including electrodes 1821, 1822, 1823, 1824, 1825, and 1826 as shown. The balloon catheter 1812 may be designed such that when deployed into a patient's body, the balloon catheter 1812 electrodes may be held in intimate contact against an endocardial surface. As an example, the balloon catheter 1812 may be inserted into a lumen, such as a pulmonary vein (PV). The balloon catheter 1812 may be inserted into the PV in a deflated state such that the balloon catheter does not occupy its maximum volume while being inserted into the PV. The balloon catheter 1812 may expand while inside the PV such that electrodes on the balloon catheter are in contact with an entire circular section of the PV. Such contact with an entire circular section of the PV, or any other lumen, may enable efficient mapping and/or ablation.

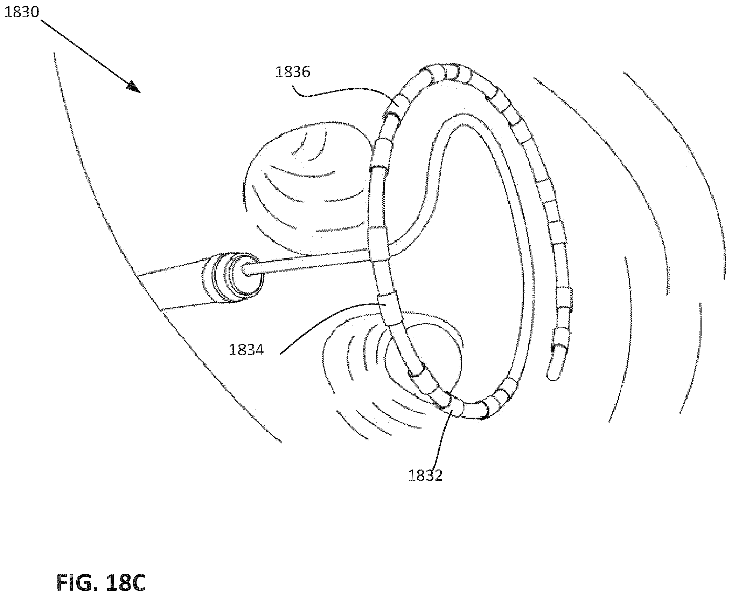

FIG. 18C shows an example of a loop catheter 1830 (also referred to as a lasso catheter) including multiple electrodes 1832, 1834, and 1836 that may be used to map a cardiac area. Loop catheter 1830 may be fully or partially elastic such that it can twist, bend, and or otherwise change its shape based on received signal and/or based on application of an external force (e.g., cardiac tissue) on the loop catheter 1830.

According to an example, a multi-electrode catheter, such as linear catheter 1802, balloon catheter 1812 and loop catheter 1830 may be advanced into a chamber of the heart. Anteroposterior (AP) and lateral fluorograms may be obtained to establish the position and orientation of each of the electrodes. Electrograms may be recorded from each of the electrodes in contact with a cardiac surface relative to a temporal reference such as the onset of the P-wave in sinus rhythm from a body surface ECG. The system, as further disclosed herein, may differentiate between those electrodes that register electrical activity and those that do not register electrical activity. The lack of registered electrical activity may be due to absence of close proximity to the endocardial wall. After initial electrograms are recorded, the multi-electrode catheter may be repositioned, and fluorograms and electrograms may be recorded again. An electrical map may be constructed from iterations of the process above..

According to an example, cardiac mapping may be generated based on detection of intracardiac electrical potential fields. A non-contact technique to simultaneously acquire a large amount of cardiac electrical information may be implemented. For example, a catheter having a distal end portion may be provided with a series of sensor electrodes distributed over its surface and connected to insulated electrical conductors for connection to signal sensing and processing means. The size and shape of the end portion may be such that the electrodes are spaced substantially away from the wall of the cardiac chamber. Intracardiac potential fields may be detected during a single cardiac beat. According to an example, the sensor electrodes may be distributed on a series of circumferences lying in planes spaced from each other. These planes may be perpendicular to the major axis of the end portion of the catheter. At least two additional electrodes may be provided adjacent at the ends of the major axis of the end portion. As a more specific example, the catheter may include four circumferences with eight electrodes spaced equiangularly on each circumference. Accordingly, in this specific implementation, the catheter may include at least 34 electrodes (32 circumferential and 2 end electrodes).

According to another example, an electrophysiological cardiac mapping system and technique based on a non-contact and non-expanded multi-electrode catheter may be implemented. Electrograms may be obtained with catheters having multiple electrodes (e.g., between 42 to 122 electrodes). According to this implementation, knowledge of the relative geometry of the probe and the endocardium may be obtained such as by an independent imaging modality such as transesophageal echocardiography. After the independent imaging, non-contact electrodes may be used to measure cardiac surface potentials and construct maps therefrom. This technique may include the following steps (after the independent imaging step): (a) measuring electrical potentials with a plurality of electrodes disposed on a probe positioned in the heart; (b) determining the geometric relationship of the probe surface and the endocardial surface; (c) generating a matrix of coefficients representing the geometric relationship of the probe surface and the endocardial surface; and (d) determining endocardial potentials based on the electrode potentials and the matrix of coefficients.

According to another example, a technique and apparatus for mapping the electrical potential distribution of a heart chamber may be implemented. An intra-cardiac multielectrode mapping catheter assembly may be inserted into a patient's heart. The mapping catheter assembly may include a multi-electrode array with an integral reference electrode, or, alternatively, a companion reference catheter. The electrodes may be deployed in the form of a substantially spherical array. The electrode array may be spatially referenced to a point on the endocardial surface by the reference electrode or by the reference catheter which is brought into contact with the endocardial surface. The electrode array catheter may carry a number of individual electrode sites (e.g., at least 24). Additionally, this example technique may be implemented with knowledge of the location of each of the electrode sites on the array, as well as a knowledge of the cardiac geometry. These locations may be determined by a technique of impedance plethysmography.

According to another example, a heart mapping catheter assembly may include an electrode array defining a number of electrode sites. The mapping catheter assembly may also include a lumen to accept a reference catheter having a distal tip electrode assembly which may be used to probe the heart wall. The mapping catheter may include a braid of insulated wires (e.g., having 24 to 64 wires in the braid), and each of the wires may be used to form electrode sites. The catheter may be readily positionable in a heart to be used to acquire electrical activity information from a first set of non-contact electrode sites and/or a second set of in-contact electrode sites.

According to another example, another catheter for mapping electrophysiological activity within the heart may be implemented. The catheter body may include a distal tip which is adapted for delivery of a stimulating pulse for pacing the heart or an ablative electrode for ablating tissue in contact with the tip. The catheter may further include at least one pair of orthogonal electrodes to generate a difference signal indicative of the local cardiac electrical activity adjacent the orthogonal electrodes.

According to another example, a process for measuring electrophysiologic data in a heart chamber may be implemented. The method may include, in part, positioning a set of active and passive electrodes into the heart, supplying current to the active electrodes, thereby generating an electric field in the heart chamber, and measuring the electric field at the passive electrode sites. The passive electrodes may be contained in an array positioned on an inflatable balloon of a balloon catheter. In embodiments, the array may have from 60 to 64 electrodes.

According to another example, cardiac mapping may be implemented using one or more ultrasound transducers. The ultrasound transducers may be inserted into a patient's heart and may collect a plurality of ultrasound slices (e.g., 2D or 3D slices) at various locations and orientations within the heart. The location and orientation of a given ultrasound transducer may be recorded and the collected ultrasound slices may be stored such that they can be displayed at a later time. One or more ultrasound slices corresponding to the position of a probe (e.g., a treatment catheter) at the later time may be displayed and the probe may be overlaid onto the one or more ultrasound slices.

According to other examples, body patches and/or body surface electrodes may be positioned on or proximate to a patient's body. A catheter with one or more electrodes may be positioned within the patient's body (e.g., within the patient's heart) and the position of the catheter may be determined by a system based on signals transmitted and received between the one or more electrodes of the catheter and the body patches and/or body surface electrodes. Additionally, the catheter electrodes may sense biometric data (e.g., LAT values) from within the body of the patient (e.g., within the heart). The biometric data may be associated with the determined position of the catheter such that a rendering of the patient's body part (e.g., heart) may be displayed and may show the biometric data overlaid on a shape of the body part, as determined by the position of the catheter.

According to exemplary embodiments, a medical procedure may be optimized by applying historical ECG data that is used to map the location of an arrhythmia to predict the location of an arrhythmia, such that the arrhythmia can be effectively treated. To clarify, numerous instances of historical ECG data used to identify an ablation that successfully treated an arrhythmia may be used as training data to generate a model in accordance with the figures as provided herein. The model may be trained such that a new ECG may be fed into the model and, based on the trained components of the models, an arrhythmia site may be identified. Such an implementation may mitigate or remove the need for manual identification of arrhythmia sites to reduce human error and may allow for a higher confidence ablation.

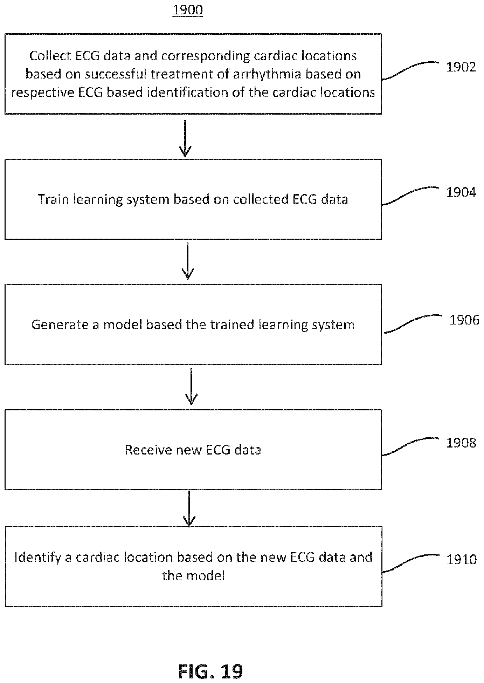

FIG. 19 is a flowchart 1900 for identifying cardiac locations based on ECG data and a model. As shown in flowchart 1900 of FIG. 19, at step 1902, historical ECG data and corresponding cardiac locations may be collected. The corresponding cardiac locations may be cardiac locations that are identified as causing an arrhythmia based on the ECG data. As further disclosed herein, the ECG data and corresponding locations collected at step 1902 may correspond to procedures where the arrhythmia is successfully treated (e.g., via ablation). Data for unsuccessful procedures may be discarded or not collected at step 1902. Alternatively, data for unsuccessful procedures may be provided as negative data and accounted for accordingly.

At step 1904 of process 1900 of FIG. 19, the ECG data and corresponding locations collected at step 1902 may be used as training data for a learning system. At step 1904, the training data may be used to train the learning system based on a given algorithm. At step 1906 of the process of 1900 of FIG. 19, the trained learning system may be used to generate a model. The model may be generated such that given new ECG inputs, the model is configured to provide new cardiac locations as outputs, the cardiac locations corresponding to predicted arrhythmia locations as predicted by the model.

At step 1908 of the process 1900 of FIG. 19, new ECG data for a patient may be received by or provided as input to the model generated at step 1906. At step 1910, the model may output an arrhythmia location for treatment (e.g., by ablation).

As shown in flowchart 1900 of FIG. 19, at step 1902, historical ECG data and corresponding cardiac locations may be collected. ECG data may be obtained by using a multiple lead ECG (e.g., a 12 lead ECG) such that cardiac signals and electronic activity of the heart are recorded and can be observed to identify conditions of the heart, such as arrhythmias. The ECG may be based on body surface (BS) electrode patches which may be placed at locations on the surface of a patient's body. Although this disclosure is generally directed to ECGs based on BS electrodes, it will be understood that intracardiac electrodes may instead be used to obtain cardiac signals to generate ECGs. The data may be obtained using one or more magnetic sensors, electrode sensors, signal filtering algorithms, advanced catheter location (ACL) technology, or the like.

As further disclosed herein, historical ECG data may be provided for a large plurality of patients. The large plurality of patients may be, for example, over 100 patients, over 1000 patients, over 10,000 patients, or the like. The number of patients whose historical ECG data is used may depend on one or more of the qualities of the ECGs, the degree of success of the corresponding ablations, the variability of ECG readings in the historical ECG data set, or the like.

According to an implementation, the ECG data provided for the large plurality of patients may correspond to procedures where an arrhythmia was successfully treated by using respective ECGs to identify the location of the arrhythmia. For example, a set of 100 ECGs may be available to train system, as further disclosed herein. From the 100 available ECGs, 70 ECGs may correspond to procedures where an ECG was used to identify the location of an arrhythmia which was then treated (e.g., via ablation) successfully. The 70 ECGs may be used as training data for the embodiments disclose herein. In contrast, the 30 ECGs that were either not used to identify a location of an arrhythmia or that identified a location of an arrhythmia, but a treatment based on the identified location was not successful, may not be included as training data.

According to implementations, ECGs may be used to identify the location of a heart condition such as an arrhythmia. As an example, a QRS complex during ventricular tachycardias (VT) may be generated from a given site of origin for focal VTs or from the exit site of a constrained diastolic isthmus during reentrant VT. The ventricular geometry and activation of a given patient can govern the ECG patterns seen in VT. As an example, a left ventricular free wall VT may show right bundle branch block (RBBB) configuration, while VT exiting from the interventricular septum or right ventricle displays left bundle branch block (LBBB) configuration. As another example, septal exits may be associated with narrower QRS complexes consistent with synchronous rather than sequential ventricular activation. As another example, basal sites may show positive precordial concordance, while negative concordance may be seen in apical sites of origin. The QRS axis may vary predominantly with shifts in exit along a superoinferior axis but also may also occur with right-left shifts. Such distinguishing attributes may be applied even in the presence of significant structural heart disease though significant scarring from prior infarction, cardiomyopathy, and congenital heart disease can reduce the precision of the ECG as a localizing tool.

As another example, anatomical variation may a factor that can cause disruption to expected patterns of ECG vectors for a given arrhythmia origin. This can arise from translational, rotational, or attitudinal shifts in the normal relationship of the heart to the chest wall, or from variations within the cardiomediastinal anatomy itself. Antiarrhythmic drugs, by affecting myocardial conduction characteristics, may be expected to affect the surface ECG appearance of VT.

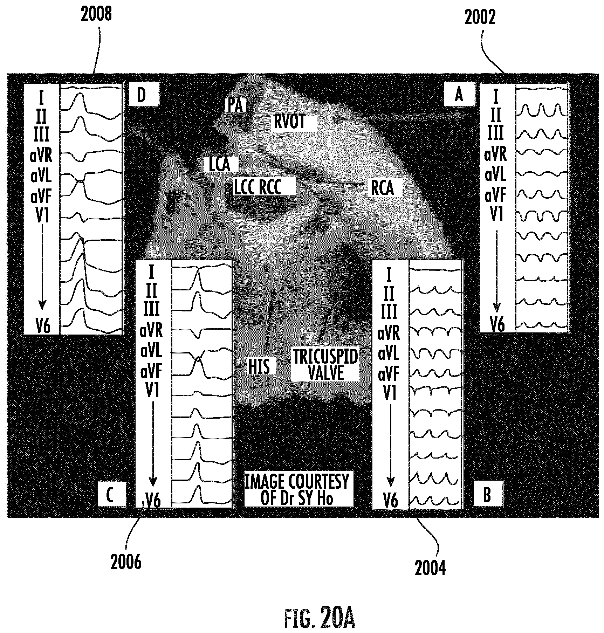

As examples of ECG data used to identify potential arrythmia locations for treatment, FIG. 20A shows an example of associating ECGs with the location of a potential arrhythmia. Specifically, FIG. 20A shows a basal view of the annular and outflow tract regions after removal of both atrial chambers. The close 3-dimensional anatomical relations of the various outflow tract and annular structures around the central fibrous cardiac skeleton are shown. The pulmonary artery (PA), right ventricular outflow tract (RVOT), left coronary cusp (LCC), right coronary cusp (RCC), left coronary artery (LCA), and right coronary artery (RCA) are shown. In FIG. 20A, 2002 shows an example surface ECG appearance of VT arising from the posterior aspect of the free wall of the RVOT. An LBBB configuration with inferior axis is seen with late precordial transition after V3 and notching in the inferior leads. The positive forces in lead I imply a posterior (or rightward) focus. At 2004 an example of the surface ECG appearance of VT with an anteroseptal RVOT origin is shown. An early precordial transition before V3 is seen with a negative lead I morphology. At 2004, the multiphasic notched configuration in lead V1 that can be seen in outflow tract VT arising from the left coronary cusp of the aortic sinus of Valsalva. At 2008 an example of VT arising from the epicardium of the left ventricular outflow tract that was mapped to the anterior interventricular vein region (where activation occurred 45 ms prior to the onset of the QRS). The ECG shows a left bundle branch block (LBBB), inferior axis configuration with broad QRS complexes of 149 ms and slurred intrinsicoid deflections.

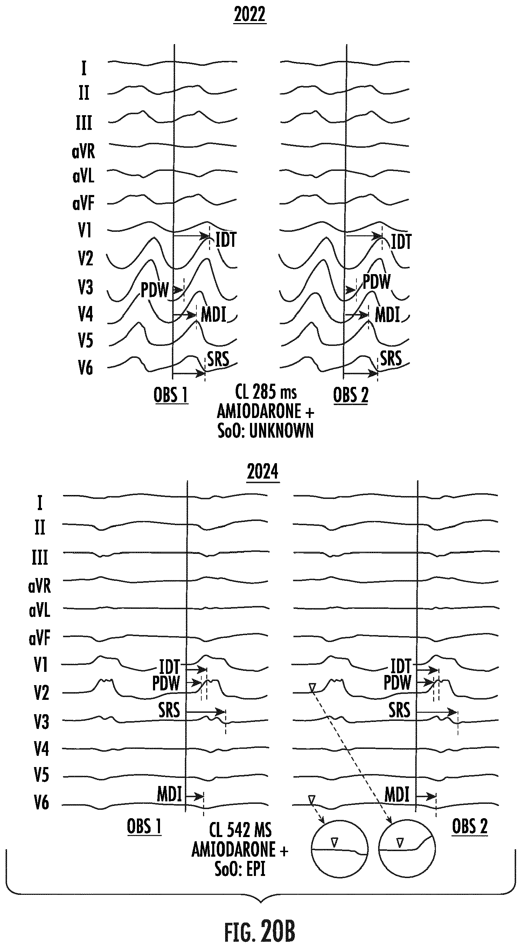

FIG. 20B shows an example of identifying ECGs criteria that can be used to identify the location of a potential arrhythmia. Specifically, FIG. 20B shows reported interval and morphological ECG criteria for identifying a left ventricular epicardial (EPI) VT site of origin are assessed by two different observers for a fast 2022 and a slow 2024 VT. Notably, for the fast VT 2022, the QRS onset is defined differently, affecting the measurement of the interval criteria. As applied in FIG. 20B, CL indicates cycle length; IDT, intrinsicoid deflection time; MDI, maximum deflection index; PDW, pseudodelta wave; and SRS, shortest RS complex.

It will be understood that FIGs. 20A and 20B are provided as examples and that potential arrhythmia sites may be identified based on ECG data using one or more other techniques. Such ECG data may be filtered based on respective successful treatments of arrhythmia such that ECG data corresponding to unsuccessful treatments is filtered out.

At step 1904 of the process 1900 of FIG. 19, the filtered ECG data may be used to train a learning system. The training may be conducted using hardware, software, and/or firmware. The training may include an analysis and correlation of the ECG data collected in step 1902. The attributes of a given ECG (e.g., such as those attributes shown in FIG. 20B) may be used to determine if a correlation or link exists between a given aspect and a corresponding cardiac arrhythmia location.

Features of the ECG data collected at step 1902 may be extracted and may include ECG attributes (e.g., such as those shown in FIG. 20B), patient history, scarring information, catheter position, ablation tags (e.g., visitags), and the like. A feature matrix may be generated based on the extracted features and the learning system may be trained at step 1904. According to an implementation, the learning system may be trained based on a machine learning algorithm, such as described herein.

The learning system may be trained using an algorithm to generate a model at step 1906. The algorithm may be, for example, a classification algorithm, a regression algorithm, a clustering algorithm, or any applicable algorithm that is able to use the ECG data to generate a model that predicts arrhythmia locations.

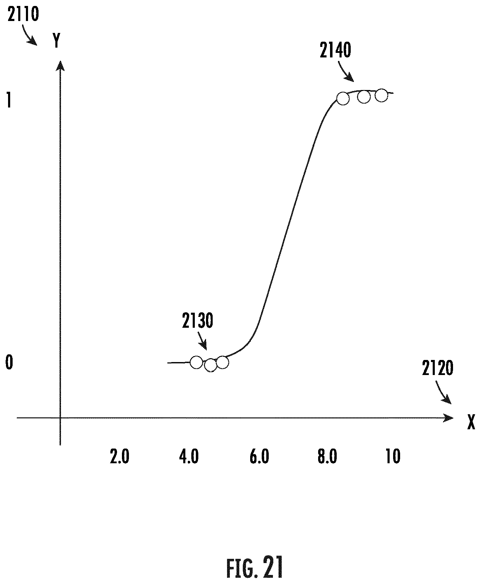

As an example, FIG. 21 shows a logistic regression diagram to predict whether certain ECG characteristics are likely to correspond to a given cardiac location with an arrhythmia. This logistic regression enables the prediction of an arrhythmia location based on ECG attributes. For example, based on plurality of QRS onsets, cycle lengths, and a maximum deflection indexes, an outcome of whether the arrhythmia originates from left atrium can be determined. The past history of the combination of given QRS onsets, cycle lengths, and a maximum deflection indexes and the relationship with an arrhythmia originating from the left atrium enables the prediction to occur. The logistic regression of FIG. 21 enables the analysis of the combination of given QRS onsets, cycle lengths, and a maximum deflection index variable 2120 to predict whether the arrhythmia originates from the left atrium with probability 2110 is defined by 0 to 1. At the low end 2130 of the S-shaped curve, a given combination of QRS onsets, cycle lengths, and a maximum deflection indexes 2120 predicts an outcome 2110 of not being in the left atrium. While at the high end 2140 of the S-shaped curve, the combination 2120 predicts an outcome 2110 of being in the left atrium.

As stated, a large number of various combinations of such features (e.g., ECG attributes, patient history, scarring information, etc.) may be used to generate a plurality of logistic regression-based outcomes, each predicting a likelihood of the location of arrhythmia in a different location based on the features. The combination of such logistic regression-based outcomes may be used to generate a logistic regression model at step 1906 that is generated based on the training of the learning system at step 1904.

It will be understood that although a logistic regression-based model is provided as an example, any applicable algorithm (e.g., classification, regression clustering, etc.) may be used to generate a respective model at step 1906.

Once ample training data (e.g., features) are used to train the learning system and generate an applicable model, the model may be used with new data. At step 1908, a new ECG may be applied to the model and the model may extract features from the new ECG and/or external features (e.g., patient history, scar information, etc.). A feature vector may be generated and input into the model generated at 1906. The model may use the feature vector as inputs (e.g., as shown in FIG. 15) and may predict an arrhythmia location based on the inputs, at step 1910. According to an implementation, multiple potential arrhythmia locations may be provided such that each potential arrhythmia location is given a score (e.g., a correlation score) which indicates a probability, as determined by the model, of the arrhythmia being at the predicted location.

According to an implementation of the disclosed subject matter, the predicted arrhythmia locations may be confirmed via a pacing procedure. A pacing procedure may be an artificially induced electronic signal that acts as the source of a potential arrhythmia.

A pacing catheter may be used at one or more of the arrhythmia locations and may induce an artificially generated electronic signal at each of the one or more of the predicted arrhythmia locations. The resulting ECG pattern may be observed to confirm whether a given location is the source of the arrhythmia. According to an implementation, a pacing catheter may be used at two or more potential arrhythmia locations that are identified by the model generated at step 1906. As disclosed herein, the model may provide two or more potential arrhythmia locations and may provide scores (e.g., correlation scores) for each of those locations. A medical professional or automated system may then use a pacing catheter at those two or more locations and observe the corresponding ECG signal patterns to confirm which of the locations corresponds to the arrhythmia location.

According to an implementation of the disclosed subject matter, patient characteristics may be used to enhance the training of the learning system at step 1902. The patient characteristics may be in addition to the ECG data that is specific to the patient. The patient characteristics may include, but are not limited to, patient age, gender, height, weight, and may also include any additional information about the patient such as disease history, cardiac structure (e.g., based on an MRI or CT scan), or the like. For example, a child's heart may be smaller than a grown adult, accordingly, identifying an arrhythmia location may be supported by such additional information.

According to this implementation, the patient characteristics may supplement the ECG data such that the learning system trained at step 1902 is trained by correlating the patient characteristics with the confirmed arrhythmia locations in addition to the ECG data correlated with the confirmed arrhythmia locations, as disclosed herein. Accordingly, the model generated at step 1906 may allow as inputs, in addition to the ECG data, the patient characteristics.

As an example, a subset of the training data collected at step 1902 may correspond to children. Accordingly, while training the learning system at 1904, the corresponding ECG data for that subset of training data may also be correlated to children. Accordingly, the model may be trained to recognize that when a child's new ECG data is provided, that the training applicable to historical children ECG data is more highly applicable than adult ECG data.

According to another implementation, the location of a catheter when ECG data is collected may be used to enhance the training of the learning system at step 1902. The location of the catheter may correspond to a location inside the heart, a cardiac chamber, a vein, and/or may correspond to proximity to tissue or another location-based characteristic.

According to this implementation, the location of the catheter may supplement the ECG data such that the learning system trained at step 1902 is trained by correlating the location of the catheter with the confirmed arrhythmia locations in addition to the ECG data correlated with the confirmed arrhythmia locations, as disclosed herein. Accordingly, the model generated at step 1906 may allow as inputs, in addition to the ECG data, the location of the catheter. Additionally, the ECG data and the location of the catheter may be used with the catheter location on the image (such as a CT/MRI/Mesh) as an additional input to the training model by normalizing the anatomical structure in such a way that the catheter locations lay in the "same areas" in the 3D Model.

As an example, a subset of the training data collected at step 1902 may correspond to a catheter located in the pulmonary vein (PV). Accordingly, while training the learning system at 1904, the corresponding ECG data for that subset of training data may also be correlated to PVs. Accordingly, the model may be trained to recognize that when a new ECG data collected in a patient's PV is provided, that the training applicable to historical PV ECG data is more highly applicable than non-PV ECG data.

As disclosed, one or more potential arrhythmia locations may be identified at step 1910. The one or more cardiac locations may be identified using a rendering or mapping (collectively, "mapping") of the heart. For example, the one or more identified cardiac locations may be displayed or highlighted on a mapping of the heart. The mapping of the heart may be generated external to the process 1900 of FIG. 19 and may be generated using location tracking using electromagnetic signals (e.g., using one or more catheters, a location pad, BS electrodes, or the like or a combination thereof).

According to an implementation of the disclosed subject matter, an improved mapping may be generated using historical improvements to mapping based on an algorithmic solution that considers the inaccuracies related to electrogram timing and presentation of complex activation patterns, as well as inaccuracies related to data projection into a rigid chamber reconstruction. Such an improved mapping is referred to herein as coherent mapping. Coherent mapping, as further disclosed herein, may be implemented improving upon traditional mapping which may be implemented using electromagnetic signals to identify catheter location (e.g., based on an electromagnetic catheter, location pads, BS patches, etc.), cardiac boundaries (e.g., tissue proximity indications), ultrasound imaging, or the like. The coherent mapping may improve upon traditional mapping based on time-based collection of data (e.g., noise, chronic signals such as respiration, inaccuracies related to electrogram timing and presentation of complex activation patterns, as well as inaccuracies related to data projection into a rigid chamber reconstruction, etc.) collected over an interval of time (e.g., 30 seconds).

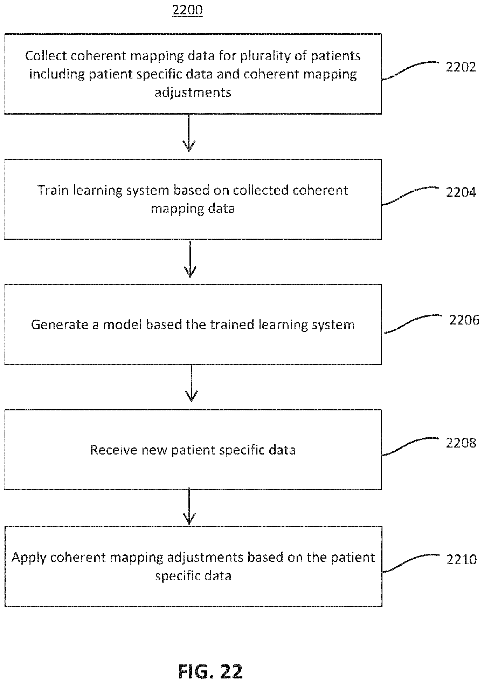

As shown in flowchart 2200 of FIG. 22, at step 2202, historical coherent mapping data may be collected. The coherent mapping data may include patient specific data and coherent mapping adjustments that are made based on the patient specific data. To clarify, the coherent mapping data enhances the traditional electromagnetic mapping, as disclosed herein.

At step 2204 of process 2200 of FIG. 22, the coherent mapping data collected at step 2202 may be used as training data for a learning system. At step 2204, the training data may be used to train the learning system based on a given algorithm. At step 2206 of the process of 2200 of FIG. 22, the trained learning system may be used to generate a model. The model may be generated such that given new patient specific data a mapping may be provided, and the model may be configured to provide coherent mapping (i.e., improved mapping) without having to re-calculate such coherent mapping data for the given new patient.

At step 2208 of the process 2200 of FIG. 22, new patient specific data may be received by or provided as input to the model generated at step 2206. At step 2210, the model may output coherent mapping data based on the new patient specific data such that the coherent mapping data is obtained without collecting it based on the patient specific data (e.g., over a time period such as 30 seconds), but rather is output by the model.

As shown at step 2202, historical coherent mapping data may be collected. The data may be collected using one or more magnetic sensors, electrode sensors, signal filtering algorithms, advanced catheter location (ACL) technology, a coherent mapping algorithm, or the like. The coherent mapping data may include patient specific data and coherent mapping adjustments that are made based on the patient specific data. The coherent mapping adjustments may be an improvement to traditional mapping and may better represent activation waves in complex substrates where the limitations presented in the introduction are the most evident. As noted herein, the coherent mapping algorithmic solution considers the inaccuracies related to electrogram timing and presentation of complex activation patterns, as well as inaccuracies related to data projection into a rigid chamber reconstruction.

LAT determination and its representation on the 3D chamber reconstruction is disclosed herein. Electrogram time annotation may be determined by a traditional mapping algorithm. According to this algorithm, LAT is determined by analysis of each bipolar electrogram with its corresponding unipolar electrograms, such that local time annotation is marked at the component with the maximal unipolar -dV/dt. However, in complex substrates with multiple potentials of relatively similar -dV/dt values, annotation of a single potential can be misleading and result in LAT inconsistencies. To solve this problem, coherent mapping may be designed to identify possible potentials of each individual electrogram and for the chamber data. Once the chamber data are obtained, the algorithm determines the most coherent global propagation under physiological conductions utilizing the possibilities for each individual electrogram.

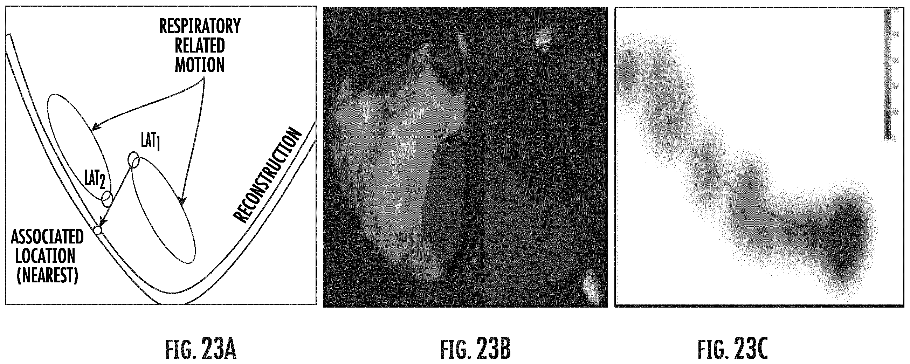

The reconstructed chamber is a static and rigid representation of a dynamic anatomy. Activation maps are created by sampling LATs from various places in the chamber. These measurements rarely fall on the rigid reconstruction because of respiratory changes, catheter mechanical effects on the chamber wall (stretching), or changes in chamber dynamics during arrhythmia. Current mapping algorithms project those points onto the nearest surface, yet the nearest location on the reconstruction is often different from the sampled location. Furthermore, using the nearest location associates samples from different locations and different LATs with a similar location of the reconstruction, as shown in FIG. 23A.

According to exemplary embodiments of the disclosed subject matter, coherent mapping data may be collected by dividing the surface into a mesh with small triangles reducing the magnitude of interpolation related to errors in projection, as shown in FIG. 23B. In addition, the effect of each individual data point on the activation map may be designed to be proportional to its distance from the nearest triangle, such that data points obtained farther away from the surface receive lower weight compared with data points obtained closer to the surface, as shown in FIG. 23C. This coherent mapping solution may reduce activation mapping inaccuracies that are related to projection of data points collected during different beats.

FIGs. 23A-C show inherent limitations related to chamber reconstruction and data projection. FIG. 23A includes a display of association of different LAT measurements with the same location. FIG. 23B shows the reconstructed chamber divided into a triangulated mesh composed of small triangles ($\approx$0.5 mm) allowing a more accurate assignment of measurements. FIG. 23C shows a sagittal section of the reconstructed chamber with a line colored based on the calculated LAT, with each individual measurement affecting the calculated LAT proportionally to its distance from it (the halo represents the fading effect with distance).

The coherent mapping algorithm assigns each triangle on the reconstructed mesh with, for example, 3 descriptors: LAT value, conduction vector, and the probability of nonconductivity. Conduction velocities are calculated using the LAT values and the known distance and direction between triangles. These descriptor values are initially known for triangles with a direct measurement but unknown for other triangles on the reconstructed mesh. To solve this problem, the following physiologically based assumptions are applied: (1) velocity continuity: in areas of conduction continuity, conduction velocity is kept as similar as possible to the neighboring triangles, and gradual changes are allowed. This relationship between triangles is performed through mathematical equations looking for the minimum mean square difference of the relations set above. The optimal solution results in minimal differences at most locations, allowing large differences in areas with enough measurements contradicting continuity; and (2) nonconduction areas are identified by multiple measurements at the same location, indicating that the electrode resides in an area with at least 2 distinct waves. In these areas, the probability of conduction slowing, or complete block, is determined by the vectors of propagation and the calculated conduction velocity. In regions with a structural obstacle for conduction, the vectors of propagation may go around the obstruction or conduct through the obstruction at a slow velocity. Conduction block was defined as a value lower than the lowest physiological conduction velocity in human atria (10 cm/s). The above criteria are used to set the probability of a triangle to be in a nonconductive area. The equations are solved until the resulting LAT, conduction velocity, and probability of nonconductivity are stabilized without further changes, representing the optimal solution.

The LAT, vector data, and identification of nonconductive or slowly conductive areas are used to generate an integrative coherent activation map. This coherent activation map is displayed as a vector map.

The techniques described above may be implemented such that coherent mapping data corresponding to a large plurality of patients (e.g., 100 patients, 1000 patients, 10,000 patents, etc.) is provided at step 2202. The coherent mapping data may include patient specific data (e.g., cardiac structure, respiratory changes, catheter mechanical effects on the chamber wall (stretching), changes in chamber dynamics during arrhythmia, etc.) and the corresponding coherent mapping adjustments made in view of the data.

At step 2204, a learning system may be trained based on the collected coherent mapping data at step 2202. The training may include extracting features from the coherent mapping data to create a feature matrix. The feature matrix may be applied to an algorithm that is used to generate a model at step 2206. The algorithm may be, for example, a classification algorithm, a regression algorithm, a clustering algorithm, or any applicable algorithm that is able to use the coherent mapping data to generate a model that predicts coherent mapping adjustments based on patient specific data for a new patient.

As an example, FIG. 13 shows an example neural network that may be implemented to train the model at step 2206. In the neural network there is an input layer represented by a plurality of inputs, such as $1410_1$ and $1410_2$. The inputs may include patient specific data (e.g., cardiac structure, respiratory changes, catheter mechanical effects on the chamber wall (stretching), changes in chamber dynamics during arrhythmia, etc.) The inputs $1410_1$, $1410_2$ are provided to a hidden layer depicted as including nodes $1420_1$, $1420_2$, $1420_3$, $1420_4$. These nodes $1420_1$, $1420_2$, $1420_3$, $1420_4$ are combined to produce an output 1430 in an output layer. The outputs, for example, may be the historical coherent mapping adjustments provided at step 2202 of FIG. 22 and may be the target that the neural network's hidden layer is aiming for. The neural network may perform processing via the hidden layer of simple processing elements, nodes $1420_1$, $1420_2$, $1420_3$, $1420_4$, which can exhibit complex global behavior, determined by the connections between the processing elements and element parameters.

The resulting model, at step 2206, may be generated such that a new set of patient specific data received at step 2208 may be applied to the model and coherent mapping adjustments may automatically be output based on the model, at 2210. Notably, by using the model the coherent mapping adjustments may be provided without having to expend the time and resources in measuring and calculating such coherent mapping adjustments during a procedure.

Once the model is created and coherent mapping adjustments provided, or as an alternative path while the coherent model is being created, the present system may also provide local noise identification using the coherent algorithm. As described herein there are several types of measurements that are used for building an anatomical cardiac model. These measurements include, for example CT, MRI, and ultrasound measurements. The measurements may be provided by an intracardiac catheter (proximity, impedance, temperature, and the like), and other sources. As is described above, a 3D model may be constructed and displayed based on these measurements of the heart (tissues and volume).

Additionally, surface ECG measurements as well as measurements from an inserted catheter are input to an existing system which calculates an average signal flow over locations and times, and displays vectors of flow in a coherent mapping / coloring. About 20 minutes of recorded signals are required as input for this system. Given the duration of the input, about 30-60 seconds are required to produce an output. Hence this process does not give real-time indication to the user.

In order to provide more real-time indications to the user, the system may provide the user some preliminary approximate coherent mapping based on partial measurements. These partial measurements may include two minutes of data instead of traditional 20, for example. The partial measurements may be provided before precise mapping is calculated, or additional thereto. Although the partial measurements may not be a precise mapping of the current patient, the partial measurements may save time by giving early feedback and guiding the user to move the catheter to a more relevant region for further measurements.

The system may provide feedback on the accuracy of a measurement taken by the catheter on a particular time point in a particular location.

The measurements taken by the catheter on a particular time point in a particular location, together with previous measurements from the patient, are used to calculate a LAT for this location as described above. Given the various sources of noise inside the body and from instruments that provide error in the LAT, providing a score, such as between 0 and 1, for example, on the accuracy of this LAT value may provide additional benefit.

As described herein, the data for each patient contains patient id and the relevant patient parameters, such as age, gender, some physical dimensions, and the like, for example. The data may further include measurements from instruments (ECG and catheter) over times and locations. The output of the system may include the existing coherent mapping described herein.

A neutral network may be trained based on this data. The input for the NN may include the patient parameters, the instrument measurements and a particular location point of interest with the LAT value calculated for this location point. The NN may output a score between 0 and 1 indicating the accuracy of the LAT value for the input location point. This output score may provide feedback to the user and may provide real-time noise filtering of measurements as input to the coherent mapping system.

On the data of a particular past patient, the known coherent mapping LAT value for each particular location point is used as the desired output ("tagging") for the supervised training of the NN. The error to minimize is the difference between the NN's output and the correct known LAT value, over the location points. Thus, no manual tagging is required. Measurements for a new patient, as well as a particular location point of interest and the calculated LAT value, may be provided to the NN, and the output is a score from 0 to 1 indicating the accuracy of the LAT value. Instead of the system which takes 30-60 sec to produce an output, data collected from previous cases may be used to train a NN, which may then produce a mapping in a much shorter amount of time. The input to the NN is the measurements taken from a patient, and the output is a complete coherent mapping.

An indirect way for approximating the mapping is to retrieve cases from previous patients that are similar to the current patient, and display some aggregation (e.g., averaging) of their coherent mappings. Each unhealthy heart behaves slightly differently, and the cardiac arrhythmia may be located in different places inside the heart. This variation may demonstrate a benefit in finding data from previous cases that are most similar to measurements from the current patient. Therefore, it may be important to retrieve similar cases given partial measurements from the current patient.

This process may be achieved by defining a distance function between representations of cases. A representation for a patient may contain the patient's measurements. The distance function may be a simple distance measure in the vector space, such as Euclidean distance, cosine similarity, or the like, for example, or a more complex function as defined by an expert. The distance function may be used to provide the most similar previous cases by using algorithms such as k-nearest neighbors described above.

Alternatively, a neural network may be trained to calculate the distance function. The input to the NN is a pair of two representations, and the output is a score between 0 and 1, indicating the similarity of the two representations. Given a dataset of N previous representations, a similarity table of size $N^2$ may be prepared, where entry (i,j) contains 0 if representations i and j are identical, 1 if they are not (or, alternatively, a score between 0 and 1 indicating their similarity). This similarity table may be filled by an expert indicating which cases are similar, or automatically (or semi-automatically) by calculating similarly between the coherent mappings associated with the representations.

Each heart is slightly different in shape. Therefore, normalization of 3D locations is required so that current measurement locations on the mesh can be faithfully compared to previous data. A 3D model of a "standard heart" may be created. A projection algorithm may be used to modify raw locations and project the raw locations onto the standard locations in the 3D model. This is done in a preprocessing stage both on the location of instrument measurements as well as on the data of the coherent mapping.

The accuracy of the system may be increased by utilizing a classifier that indicates which class of cardiac abnormality (e.g. Flutter, VT, Micro-orientry, etc.) is relevant for the current patient. This classifier may be used in a preprocessing stage in the system. Specifically, both previous data and current patient data may be classified to detect the relevant class. Instead of creating just one model to account for all cases, a separate model may be constructed for each class. Each model may be specialized on just one (or a few) particular class to increase the accuracy of results.

Alternatively, data from previous patients may contain a relevant classification as assigned by a physician. Supervised learning may be used to build a classifier based on this data where the input is the patient's data, and the output is the assigned abnormality class.

Even after the system is ready and is deployed in hospitals, additional data may be accumulated. The accumulated data may be added to the training dataset, and the system may be re-trained, to continually improve its accuracy.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random-access memory (RAM), a register, cache memory, semiconductor memory devices, magnetic media such as internal hard disks and removable disks, magneto-optical media, and optical media such as CD-ROM disks, and digital versatile disks (DVDs). A processor in association with software may be used to implement a radio frequency transceiver for use in a WTRU, UE, terminal, base station, RNC, or any host computer.

**Claims**

1. A system for automatically detecting arrhythmia locations, comprising:

   a plurality of body surface electrodes configured to sense electrocardiogram (ECG) data;
   a display; and
   a processor comprising a neural network and configured to:

      receive a plurality of historical ECG data and corresponding arrhythmia locations determined based on each of the plurality of historical ECG data;
      train a learning system based on the plurality of historical ECG data and corresponding arrhythmia locations;
      generate a model based on the learning system;
      receive new ECG data from the plurality of body surface electrodes;
      provide a new arrhythmia location based on the new ECG data and the model; and
      render the new arrhythmia location on the display.

2. The system of claim 1, wherein the received plurality of historical ECG data and corresponding arrhythmia locations correspond to successfully treated arrhythmias at the corresponding arrhythmia locations.

3. The system of claim 1, further comprising an ablation catheter.

4. The system of claim 3, wherein the ablation catheter is located at the new arrhythmia location and configured to treat the arrhythmia.

5. The system of claim 1, wherein the learning system is trained using at least one selected from the group consisting of a classification, a regression and a clustering algorithm.

6. The system of claim 1, wherein the processor comprising a neural network is further configured to :

   receive patient characteristics;
   train the learning system based on the patient characteristics; and
   generate the model based on the further trained learning system.

7. The system of claim 1, wherein the processor comprising a neural network is further configured to:

   receive catheter location data;
   train the learning system based on the catheter location data; and
   generate the model based on the further trained learning system.

8. The system of claim 1, wherein the processor comprising a neural network is further configured to assign a score to at least one of the corresponding arrhythmia locations, wherein the score corresponds to a noise probability of the at least one of the corresponding arrhythmia locations.

9. The system of claim 8 wherein the score is within a range from 0 to 1.

10. The system of claim 8 wherein the processor comprising a neural network is further configured to filter out locations with a score of 0.

11. A method for generating an arrhythmia prediction model, the method comprising:

receiving a plurality of historical ECG data and corresponding arrhythmia locations determined based on each of the plurality of historical ECG data;
training a learning system based on a first set of historical ECG data from the plurality of historical ECG data and corresponding arrhythmia locations such that combinations of ECG attributes from the ECG are correlated with a first set of the corresponding arrhythmia locations;
updating the learning system based on a second set of historical ECG data from the plurality of historical ECG data and corresponding arrhythmia locations such that the combinations of ECG attributes from the ECG are correlated with a second set of corresponding arrhythmia locations; and
generating a model based on the first set of the corresponding arrhythmia locations and the second set of corresponding arrhythmia locations.

12. The method of claim 11 wherein the second set of corresponding arrhythmia locations are improved first set of corresponding arrhythmia locations.

13. The method of claim 11, further comprising assigning a score to at least one of the corresponding arrhythmia locations, wherein the score corresponds to a noise probability of the at least one of the corresponding arrhythmia locations.

14. The method of claim 13 wherein the score is within a range from 0 to 1.

15. The method of claim 13 further comprising filtering out locations with a score of 0.

16. A system for automatically applying coherent mapping, comprising:

an intrabody catheter configured to detect location within a heart;
a processor comprising a neural network and configured to:

receive a plurality of historical coherent mapping data for a plurality of patients, the historical coherent mapping data comprising patient specific data and a plurality of coherent mapping adjustments;
train a learning system based on the historical coherent mapping data;
generate a model based on the learning system;
receive new mapping data using the intrabody catheter; and
provide a new coherent mapping adjustment based on the new mapping data and the model.

17. The system of claim 16, wherein the coherent mapping adjustments comprise at least any one or a combination of respiratory changes, catheter mechanical effects on a chamber wall, and changes in chamber dynamics during arrhythmia.

18. The system of claim 16, wherein the new mapping data comprises inputs to the model and the new coherent mapping adjustments are an output of the model.

19. The system of claim 16, wherein the processor comprising a neural network is further configured to assign a score to at least a portion of the new mapping data, wherein the score corresponds to a noise probability of the at least a portion of the new mapping data.

20. The system of claim 19 wherein the score is within a range from 0 to 1, and wherein the processor comprising a neural network is further configured to filter out at least one new coherent mapping adjustment of the model as a result of a score of 0.

100

106 LOCAL COMPUTING DEVICE

110 NETWORK

120 NETWORK

108 REMOTE COMPUTING SYSTEM

104

PATIENT

MONITORING AND PROCESSING APPARATUS

SENSOR — 112

PROCESSOR — 114

UI SENSOR — 116

102

MEMORY — 118

TRANSCEIVER — 122

FIG. 1

FIG. 2

FIG. 3

EP 3 928 702 A2

**400**

**420**

**410**

Training the Machine

**450**

Hardware

**430**

Data

Building a Model

**440**

Predicting Outcome

# FIG. 4

EP 3 928 702 A2

**500**

COLLECT DATA FROM HARDWARE ← **510**

TRAIN A MACHINE ON THE HARDWARE ← **520**

BUILD A MODEL ← **530**

PREDICT OUTCOMES OF THE HARDWARE ← **540**

FIG. 5

610 →

| Weather | Play |
|---|---|
| Sunny | No |
| Overcast | Yes |
| Rainy | Yes |
| Sunny | Yes |
| Sunny | Yes |
| Overcast | Yes |
| Rainy | No |
| Rainy | No |
| Sunny | Yes |
| Rainy | Yes |
| Sunny | No |
| Overcast | Yes |
| Overcast | Yes |
| Rainy | No |

620 →

**Frequency Table**

| Weather | No | Yes |
|---|---|---|
| Overcast | | 4 |
| Rainy | 3 | 2 |
| Sunny | 2 | 3 |
| Grand Total | 5 | 9 |

630 →

**Likelihood Table**

| Weather | No | Yes | | |
|---|---|---|---|---|
| Overcast | | 4 | =4/14 | 0.29 |
| Rainy | 3 | 2 | =5/14 | 0.36 |
| Sunny | 2 | 3 | =5/14 | 0.36 |
| All | 5 | 9 | | |
| | =5/14 | =9/14 | | |
| | 0.36 | 0.64 | | |

## FIG. 6

FIG. 7

FIG. 8

FIG. 9

EP 3 928 702 A2

FIG. 10

FIG. 11

FIG. 12

Bagging - Parallel

Boosting - Sequential

FIG. 13

FIG. 14

FIG. 15

EP 3 928 702 A2

FIG. 16A

**FIG. 16B**

1600

1612

FIG. 16C

EPICARDIAL VOLTAGE MAP VS POTENTIAL DURATION MAP
IN LEFT VENTRICULAR NON-COMPACTION CARDIOMYOPATHY

FIG. 16D

FIG. 17

FIG. 18A

FIG. 18B

FIG. 18C

1900

Collect ECG data and corresponding cardiac locations
based on successful treatment of arrhythmia based on
respective ECG based identification of the cardiac locations — 1902

Train learning system based on collected ECG data — 1904

Generate a model based the trained learning system — 1906

Receive new ECG data — 1908

Identify a cardiac location based on the new ECG data and
the model — 1910

FIG. 19

FIG. 20A

FIG. 20B

FIG. 21

EP 3 928 702 A2

2200

```
┌─────────────────────────────────────────┐
│ Collect coherent mapping data for        │
│ plurality of patients including patient  │──── 2202
│ specific data and coherent mapping       │
│ adjustments                              │
└─────────────────────────────────────────┘
                    │
                    ▼
┌─────────────────────────────────────────┐
│ Train learning system based on collected │──── 2204
│ coherent mapping data                    │
└─────────────────────────────────────────┘
                    │
                    ▼
┌─────────────────────────────────────────┐
│ Generate a model based the trained       │──── 2206
│ learning system                          │
└─────────────────────────────────────────┘
                    │
                    ▼
┌─────────────────────────────────────────┐
│ Receive new patient specific data        │──── 2208
└─────────────────────────────────────────┘
                    │
                    ▼
┌─────────────────────────────────────────┐
│ Apply coherent mapping adjustments based │──── 2210
│ on the patient specific data             │
└─────────────────────────────────────────┘
```

**FIG. 22**

RESPIRATORY
RELATED
MOTION

LAT₁

LAT₂

RECONSTRUCTION

ASSOCIATED
LOCATION
(NEAREST)

FIG. 23A

FIG. 23B

FIG. 23C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63034508